(19) **Europäisches Patentamt / European Patent Office / Office européen des brevets**

(11) **EP 3 717 664 B1**

(12) **EUROPEAN PATENT SPECIFICATION**

(45) Date of publication and mention of the grant of the patent:
**29.06.2022 Bulletin 2022/26**

(21) Application number: **18804351.7**

(22) Date of filing: **26.11.2018**

(51) International Patent Classification (IPC):
**C12Q 1/6876** (2018.01)

(52) Cooperative Patent Classification (CPC):
**C12Q 1/6876;** C12Q 2600/124; C12Q 2600/158

(86) International application number:
**PCT/EP2018/082592**

(87) International publication number:
**WO 2019/102007 (31.05.2019 Gazette 2019/22)**

(54) **GENETIC BIOMARKER PROFILES FOR ENDURANCE SPORT SUITABILITY**

GENETISCHE BIOMARKERPROFILE FÜR AUSDAUERSPORTTAUGLICHKEIT

PROFILS DE BIOMARQUEURS GÉNÉTIQUES POUR ADÉQUATION DE SPORT D'ENDURANCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2017 EP 17382799**

(43) Date of publication of application:
**07.10.2020 Bulletin 2020/41**

(73) Proprietors:
- **Universitat Politècnica De Catalunya**
  **08034 Barcelona (ES)**
- **Fundació Institut de recerca de l'Hospital de la Santa Creu i Sant Pau**
  **08025 Barcelona (ES)**
- **Summit 2014, S.L.**
  **08540 Centelles, Barcelona (ES)**

(72) Inventors:
- **PERERA LLUNA, Alexandre**
  **08028 Vilanova I La Geltru (ES)**
- **MAQUEDA GONZÁLEZ, María**
  **08902 Hospitalet De Llobregat (ES)**
- **ROCA RODRÍGUEZ, Emma**
  **25721 Tallo (ES)**
- **SORIA FERNÁNDEZ, José Manuel**
  **08031 Barcelona (ES)**

(74) Representative: **Juncosa Miró, Jaime Torner, Juncosa i Associats, S.L.**
**C/Pau Claris, 108, 1r 1a**
**08009 Barcelona (ES)**

(56) References cited:
- **E BARREY ET AL: "Gene expression profiling in blood cells of endurance horses completing competition or disqualified due to metabolic disorder", EQUINE VETERINARY JOURNAL. SUPPLEMENT, vol. 36, no. 36, 1 August 2006 (2006-08-01), pages 43-49, XP055442497, England**
- **GRAZYNA JANIKOWSKA ET AL: "Differences in echocardiography, blood pressure, stroke volume, maximal power and profile of genes related to cardiac hypertrophy in elite road cyclists", ADVANCES IN CLINICAL AND EXPERIMENTAL MEDICINE, vol. 26, no. 6, 29 September 2017 (2017-09-29), pages 999-1005, XP055442868, PL ISSN: 1899-5276, DOI: 10.17219/acem/63031**
- **M. YOSHIOKA: "Serial analysis of gene expression in the skeletal muscle of endurance athletes compared to sedentary men", THE FASEB JOURNAL, vol. 17, no. 13, 1 October 2003 (2003-10-01), pages 1812-1819, XP055441878, US ISSN: 0892-6638, DOI: 10.1096/fj.02-1200com cited in the application**
- **LINDA MUSTELIN ET AL: "Acquired obesity and poor physical fitness impair expression of genes of mitochondrial oxidative phosphorylation in monozygotic twins discordant for obesity", AMERICAN JOURNAL OF PHYSIOLOGY: ENDOCRINOLOGY AND METABOLISM., vol. 295, no. 1, 6 May 2008 (2008-05-06), pages E148-E154, XP055442359, US ISSN: 0193-1849, DOI: 10.1152/ajpendo.00580.2007**

- **SOPHIE L. WARDLE ET AL: "Plasma MicroRNA Levels Differ between Endurance and Strength Athletes", PLOS ONE, vol. 10, no. 4, 16 April 2015 (2015-04-16) , page e0122107, XP055442873, DOI: 10.1371/journal.pone.0122107**
- **ZEIBIG J ET AL: "Do blood cells mimic gene expression profile alterations known to occur in muscular adaptation to endurance training ?", EUROPEAN JOURNAL OF APPLIED PHYSIOLOGY, SPRINGER, BERLIN, DE, vol. 95, no. 1, 1 September 2005 (2005-09-01), pages 96-104, XP019343540, ISSN: 1439-6327, DOI: 10.1007/S00421-005-1334-3**
- **PETER H. CONNOLLY ET AL: "Effects of exercise on gene expression in human peripheral blood mononuclear cells", JOURNAL OF APPLIED PHYSIOLOGY., vol. 97, no. 4, 11 June 2004 (2004-06-11), pages 1461-1469, XP055442289, US ISSN: 8750-7587, DOI: 10.1152/japplphysiol.00316.2004**

**Description**

**FIELD OF THE INVENTION**

The invention relates to the field of gene expression analysis of athletes. In particular, the invention relates to methods for determining the fitness of a subject based on the expression level of one or more genes.

**BACKGROUND OF THE INVENTION**

The level of strength and endurance defines the physical performance of an individual, encompassing a series of traits that determine it, such as the rate of metabolism or specific measures of cardiovascular capacity. These features are affected by both environmental (e.g. training) and genetic factors. The main approaches from a genetic point of view are the identification of the natural talent of an athlete together with the development of individualized training programs to maximize their potential. Most of the studies in this field focus on characterizing the phenotype of the physical performance and fitness of an individual through genome-wide association studies (GWAS). In these studies one or more genetic variations (Single Nucleotide Polymorphism, SNP) along the human genome are analyzed and associated with a certain trait observable in said phenotype. In this line, a list of genes obtained from the collection of a whole series of studies related to the practice of a particular type of exercise or training, comparing active athletes or active individuals with sedentary people, was published. Specifically, this list consists of 140 genes, 11 of which are mitochondrial (Bray, M. S. et al, Medicine & Science in Sports & Exercise, 2009, 41 (1): 34-72).

Gene expression studies related to the physical performance focus on the analysis of the behavior of the skeletal muscle. In this way, experiments have been carried out to determine the existence of differential gene expression among individuals with a certain level of training and sedentary individuals (Yoshioka, M. et al, FASEB Journal 2003, 17(13): 1812-19), leading to the identification of possible candidate genes responsible for performance improvement. In the same line, endurance athletes were compared with strength athletes (Stepto, N. K. et al, Medicine & Science in Sports & Exercise, 2009, 41 (3): 546-65).

Barrey et al. "Gene expression profiling in blood cells of endurance horses completing competition or disqualified due to metabolic disorder" shows that genes are modulated in leucocytes in relationship with performance and clinical status of the horses. In addition, gene expression in leucocytes, haematological and biochemical parameters were compared between successful and disqualified endurance horses.

Grazyna Janikowska et al. "Differences in echocardiography, blood pressure, stroke volume, maximal power and profile of genes related to cardiac hypertrophy in elite road cyclists" discloses that regular and moderate exercise is beneficial for improving the efficiency of the heart, but high-intensity physical activity may result in cardiac changes. This study focuses on the identification of the differences in echocardiography and blood variables before exercise, as well as the genes associated with cardiac hypertrophy at rest and in response to graded exercise test. The study group was made up of 28 road cyclists. Echocardiographic parameters and blood pressure were measured before exercise tests (N = 28). Blood samples were collected at rest, at maximal exercise intensity in a graded bicycle test and after 15 min of recovery; afterwards, blood morphology was estimated and RNA was isolated. Analysis of the expression profile of genes was performed for randomly selected road cyclists using the microarray method.

According to Yoshioka et al. "Serial analysis of gene expression in the skeletal muscle of endurance athletes compared to sedentary men", physical exercise produces several adaptive changes in skeletal muscle. However, the molecular mechanisms of these effects are poorly understood. This study performs serial analysis of gene expression (SAGE) to quantify the global gene expression profile in sedentary and endurance-trained muscle. A total of 10869 SAGE tags was sequenced and represented 4727 genes. The genes most expressed in muscle are mainly involved in contraction and energy metabolism. Thirty-three genes were differentially expressed between endurance athletes and sedentary individuals. Four genes such as myosin binding protein C fasttype, glycogen phosphorylase, and pyruvate kinase were expressed less in endurance athletes, whereas eight genes coding for expressed sequence tag similar to (EST) crystallin alpha B, EST myosin light chain 2, EST surfactant pulmonary-associated protein A1, EST thrombospondin, EST fructose-bisphosphate aldolase A, EST cytochrome oxidase 1, NADH dehydrogenase 3, and G8 protein were up-regulated. Most of the up-regulated tags corresponded to novel genes. On the other hand, different isoforms of fructose-bisphosphate aldolase A were also differentially expressed.

Mustelin et al. "Acquired obesity and poor physical fitness impair expression of genes of mitochondrial oxidative phosphorylation in monozygotic twins discordant for obesity" discloses a study showing that, independent of genetic factors, obesity is associated with poor fitness, low insulin sensitivity, and decreased transcript levels of genes involved in mitochondrial oxidative phosphorylation. This conclusion is based on a study of a group of genetically identical MZ twin pairs discordant or concordant for BMI. MZ twin pairs discordant for obesity offer a unique opportunity to study effects of acquired obesity independently of confounding genetic influences while matching for age, sex, and many socioeconomic, childhood, and familial factors.

Finally, there are combined studies of gene expression and association focused on improving aspects related to the health of the individual. Examples are those that have as an objective increasing the aerobic capacity (maximal VO$_2$) associated with a greater risk of cardiovascular disease. Timmons et al. (Timmons, J. A. et al, Journal of Applied Physiology, 1985, 108: 1487-96) monitors the increase of VO$_2$ max along a resistance training of the patient. US2014/0094381 discloses the results in this regard.

However, a specific method of analysis of the expression profile of an individual in order to identify a group of genes that may be related to the fitness state has not been carried out to date.

Therefore, there is a need in the art of methods for identifying the fitness of an individual based on their genetic profile.

**BRIEF DESCRIPTION OF THE INVENTION**

The authors of the present invention have surprisingly found that the expression levels of a small set of genes have prediction value for the fitness of an individual related to his level of training. In particular, the inventors have developed a predictive model of the fitness of a subject based on the expression levels of a number of genes from pre-race and post-race samples from Ultra-Marathon Trail (UMT) runners (see examples).

The invention is set out in the appended set of claims.

Thus, in a first aspect, the invention relates to an *in vitro* method for determining the fitness of a subject that comprises determining the expression level of at least one gene selected from the group consisting of GZMK, SNORA38B, and IGF2R in a sample from said subject, said sample selected from blood, blood plasma and serum.

In a second aspect, the invention relates to an *in vitro* method for determining the fitness of a subject that comprises:

(i) determining the expression levels of one or more genes selected from the group consisting of GZMK, SNORA38B, and IGF2R, in a sample from said subject, said sample selected from blood, blood plasma and serum, and
(ii) comparing the expression levels obtained in (i) with a reference value, wherein

if the reference value is obtained from one or more elite/professional subjects,

- an increased or equal expression level of at least one of GZMK and SNORA38B genes compared to the reference value, and/or a decreased or equal expression level of IGF2R, gene compared to the reference value indicates that the fitness of the subject is elite/professional, and
- a decreased expression level of at least one of GZMK and SNORA38B genes compared to the reference value, and/or an increased expression level of IGF2R gene compared to the reference value indicates that the fitness of the subject is active/acceptable or passive/sedentary

if the reference value is obtained from one or more active or acceptable subjects,

- a decreased or equal expression level of at least one of GZMK and SNORA38B genes compared to the reference value, and/or an increased or equal expression level of IGF2R gene compared to the reference value indicates that the fitness of the subject is active/acceptable or passive/sedentary, and
- an increased expression level of at least one of GZMK and SNORA38B genes compared to the reference value, and/or a decreased expression level of IGF2R gene compared to the reference value indicates that the fitness of the subject is elite/professional and

if the reference value is obtained from one or more passive/sedentary subjects,

- an increased expression level of at least one of GZMK and SNORA38B genes compared to the reference value, and/or a decreased expression level of IGF2R gene compared to the reference value indicates that the fitness of the subject is elite/professional or active/acceptable, and
- a decreased or equal expression level of at least one of GZMK and SNORA38B genes compared to the reference value, and/or an increased or equal expression level of IGF2R gene compared to the reference value indicates that the fitness of the subject is passive/sedentary.

In a third aspect, the invention relates to an *in vitro* method for selecting a subject as having a fitness level suitable to participate in an endurance sport that comprises determining the fitness of the subject using the method according to the first aspect or the method according to any the second aspect, wherein if the fitness level is active/acceptable or elite/professional the subject has a fitness level suitable to participate in an endurance sport.

In a fourth aspect, the invention relates to an *in vitro* method for monitoring the response of a subject to a training program aimed at improving fitness of a subject that comprises

(i) determining the expression levels of one or more genes selected from the group consisting of GZMK, SNORA38B, and IGF2R, in a sample from said subject, said sample selected from blood, blood plasma and serum, during or after the training program, and
(ii) comparing the expression levels obtained in (i) with the levels of said gene(s) in a sample from the same subject, said sample selected from blood, blood plasma and serum, in a previous point of time,

wherein an increased expression level of at least one of GZMK and SNORA38B genes, and/or a decreased expression level of IGF2R gene compared to the levels in the sample from a previous point of time indicates that the subject has a good response to training, and
wherein a decreased expression level or a lack of change of at least one of GZMK and SNORA38B genes, and/or an increased expression level or a lack of change of IGF2R gene compared to the levels in the sample from a previous point of time indicates that the subject has a bad response to training.

In a fifth aspect, the invention relates to an *in vitro* method for selecting a personalized training program aimed at improving fitness of a subject that comprises

(i) determining the expression levels of one or more genes selected from the group consisting of GZMK, SNORA38B, and IGF2R, in a sample from said subject, said sample selected from blood, blood plasma and serum, during or after the training program under test, and
(ii) comparing the expression levels obtained in (i) with the levels of a sample from the same subject, said sample selected from blood, blood plasma and serum, before starting the training program under test,

wherein the training program under test is selected if there is an increased expression level of at least one of GZMK and SNORA38B genes, and/or a decreased expression level of IGF2R gene compared to the level of expression before starting the training program, and
wherein the training program under test is not selected if there is a decreased expression level or a lack of change of at least one of GZMK and SNORA38B genes, and/or an increased expression level or a lack of change of IGF2R gene compared to the level of expression before starting the training program.

Not forming part of the invention, a kit comprising a set of reagents capable of determining the expression levels of at least two genes selected from the group consisting of GZMK, SNORA38B, and IGF2R is disclosed. Said reagents are oligonucleotides that specifically hybridize with the transcriptional product of the genes or compounds that specifically bind to the protein encoded by the genes; and wherein said reagents comprise at least the 10 % of the total amount of reagents for assaying gene expression markers forming the kit.

Neither forming part of the invention the use of a kit as defined above is disclosed; or of a reagent capable of determining the expression levels of at least one gene selected from the group consisting of GZMK, SNORA38B, and IGF2R, and optionally of a set of reagents capable of determining the expression levels of one or more of LILRA6, KLF7, SPATA2, RAB19, ATP6V0D1 SPRY3, GPR97, SNORD116-24, SLC43A2, ZSWIM6, and LOC399715,for determining the fitness of a subject or for selecting a subject as having a fitness level suitable to participate in an endurance sport or for monitoring the response to training of a subject during an endurance exercise training, or for selecting a personalized training program aimed at improving fitness of a subject, wherein said reagents are oligonucleotides that specifically hybridize with the transcriptional product of the gene or compounds that specifically bind to the protein encoded by the gene.

In sixth aspect, the invention relates to a computer-implemented method for determining the fitness of a subject according to any one of the first and second aspects, or for selecting a subject as having a fitness level suitable to participate in an endurance sport according to the third aspect, or for monitoring the response to training of a subject during an endurance exercise training according to the fourth aspect, or for selecting a personalized training program aimed at improving fitness of a subject according to the fifth aspect, which computer-implemented method comprises:

- collection of the data of the expression level of the genes determined in methods of aspects first to fifth;
- analyzing the collected data by (a) calculating a predictive factor by summing the expression levels of said genes, wherein said expression levels are corrected by a particular coefficient for each of the genes, wherein said predictive factor is indicative of the fitness of the subject, and wherein said coefficient for each of the genes is a coefficient as shown in columns 4 and 5 of Table 3.; or by (b) comparing the expression levels obtained with a reference value; or by (c) comparing the expression levels obtained with the levels of said genes in a previous point of time; or by (d) comparing the expression levels with the levels of said genes, before starting a training program under test; and
- providing a result of the analysis.

In a seventh aspect, the invention relates to a computer program for executing the computer-implemented method according to the sixth aspect.

This description also discloses an *in vitro* method for determining the fitness of a subject that comprises:

(i) determining the expression level of at least one gene selected from the group consisting of GZMK, SNORA38B, IGF2R, LILRA6, KLF7, SPATA2, RAB19, and ATP6V0D1 in a sample from said subject, and
(ii) calculating a predictive factor by summing the expression levels of said genes, wherein said expression levels are corrected by a particular coefficient for each of the genes,

wherein said predictive factor is indicative of the fitness of the subject.

The description also discloses an *in vitro* method for determining the fitness of a subject that comprises:

(i) determining the expression levels of one or more genes selected from the group consisting of GZMK, SNORA38B, IGF2R, LILRA6, KLF7, SPATA2, RAB19, and ATP6V0D1 in a sample from said subject, and
(ii) comparing the expression levels obtained in (i) with a reference value, wherein

if the reference value is obtained from at least one subject that practices more than 10 hours a week of physical exercise, termed elite/professional subject,

- an increased or equal expression level of at least one of GZMK and SNORA38B genes compared to the reference value, and/or a decreased or equal expression level of at least one of IGF2R, LILRA6, KLF7, SPATA2, RAB19 and ATP6V0D1 genes compared to the reference value indicates that the fitness of the subject is elite/professional, and
- a decreased expression level of at least one of GZMK and SNORA38B genes compared to the reference value, and/or an increased expression level of at least one of IGF2R, LILRA6, KLF7, SPATA2, RAB19 and ATP6V0D1 genes compared to the reference value indicates that the fitness of the subject is active/acceptable or passive/sedentary

if the reference value is obtained from at least one subject that practices between three and ten hours a week of physical exercise, termed active or acceptable subject,

- a decreased or equal expression level of at least one of GZMK and SNORA38B genes compared to the reference value, and/or an increased or equal expression level of at least one of IGF2R, LILRA6, KLF7, SPATA2, RAB19 and ATP6V0D1 genes compared to the reference value indicates that the fitness of the subject is active/acceptable or passive/sedentary, and
- an increased expression level of at least one of GZMK and SNORA38B genes compared to the reference value, and/or a decreased expression level of at least one of IGF2R, LILRA6, KLF7, SPATA2, RAB19 and ATP6V0D1 genes compared to the reference value indicates that the fitness of the subject is elite/professional, and

if the reference value is obtained from at least one subject that practices less than three hours of physical exercise a week, termed passive/sedentary subject,

- an increased expression level of at least one of GZMK and SNORA38B genes compared to the reference value, and/or a decreased expression level of at least one of IGF2R, LILRA6, KLF7, SPATA2, RAB19 and ATP6V0D1 genes compared to the reference value indicates that the fitness of the subject is elite/professional or active/acceptable, and
- a decreased or equal expression level of at least one of GZMK and SNORA38B genes compared to the reference value, and/or an increased or equal expression level of at least one of IGF2R, LILRA6, KLF7, SPATA2, RAB19 and ATP6V0D1 genes compared to the reference value indicates that the fitness of the subject is passive/sedentary.

The description also discloses an *in vitro* method for selecting a subject as having a fitness level suitable to participate in an endurance sport that comprises determining the fitness of the subject using the method of the first aspect or the method of the second aspect, wherein if the fitness level is active/acceptable or elite/professional the subject has a fitness level suitable to participate in an endurance sport.

This description also discloses an *in vitro* method for monitoring the response of a subject to a training program aimed at improving fitness of a subject that comprises

(i) determining the expression levels of one or more genes selected from the group consisting of GZMK, SNORA38B, IGF2R, LILRA6, KLF7, SPATA2, RAB19 and ATP6V0D1 in a sample from said subject during or after the training program, and

(ii) comparing the expression levels obtained in (i) with the levels of said gene(s) in a sample from the same subject in a previous point of time,

wherein an increased expression level of at least one of GZMK and SNORA38B genes, and/or a decreased expression level of at least one of IGF2R, LILRA6, KLF7, SPATA2, RAB19 and ATP6V0D1 genes compared to the levels in the sample from a previous point of time indicates that the subject has a good response to training, and

wherein a decreased expression level or a lack of change of at least one of GZMK and SNORA38B genes, and/or an increased expression level or a lack of change of at least one of IGF2R, LILRA6, KLF7, SPATA2, RAB19 and ATP6V0D1 genes compared to the levels in the sample from a previous point of time indicates that the subject has a bad response to training.

It is also disclosed an *in vitro* method for selecting a personalized training program aimed at improving fitness of a subject that comprises

(i) determining the expression levels of one or more genes selected from the group consisting of GZMK, SNORA38B, IGF2R, LILRA6, KLF7, SPATA2, RAB19 and ATP6V0D1 in a sample from said subject during or after the training program under test, and

(ii) comparing the expression levels obtained in (i) with the levels of a sample from the same subject before starting the training program under test,

wherein the training program under test is selected if there is an increased expression level of at least one of GZMK and SNORA38B genes, and/or a decreased expression level of at least one of IGF2R, LILRA6, KLF7, SPATA2, RAB19 and ATP6V0D1 genes compared to the level of expression before starting the training program, and

wherein the training program under test is not selected if there is a decreased expression level or a lack of change of at least one of GZMK and SNORA38B genes, and/or an increased expression level or a lack of change of at least one of IGF2R, LILRA6, KLF7, SPATA2, RAB19 and ATP6V0D1 genes compared to the level of expression before starting the training program.

Not forming part of the invention, the description also discloses a kit comprising a set of reagents capable of determining the expression levels of at least two genes selected from the group consisting of GZMK, SNORA38B, IGF2R, LILRA6, KLF7, SPATA2, RAB19 and ATP6V0D1.

Neither forming part of the invention, the description also discloses the use of a reagent capable of determining the expression levels of at least one gene selected from the group consisting of GZMK, SNORA38B, IGF2R, LILRA6, KLF7, SPATA2, RAB19, ATP6V0D1 SPRY3, GPR97, SNORD116-24, SLC43A2, ZSWIM6, and LOC399715 or of the above-cited kit for determining the fitness of a subject or for selecting a subject as having a fitness level suitable to participate in an endurance sport.

The description also discloses a computer program for executing a method according to any aspects first to third.

The description also discloses a computer-implemented method for determining the fitness of a subject according to the first or second aspect or for selecting a subject as having a fitness level suitable to participate in an endurance sport according to the third aspect.

**BRIEF DESCRIPTION OF THE FIGURES**

**Figure 1.** General overview of the pipeline for obtaining the predictive model.
**Figure 2.** Detailed workflow for the predictive model.
**Figure 3**: PCA (principal component analysis) over top 100 TCs (transcript clusters) expression values from the total 268 TCs ranked through the interaction effect in LM (linear regression model).
**Figure 4**: First PC scores of samples with pre and post data. Samples are labelled with subject training level and race completed distance.
**Figure 5**: PRESS (Predicted Residual Sum of Squares) evolution of the PLSR (Partial Least Squares Regression) models iteratively obtained as genes were added to them. PRESS values are shown for all available latent variables in each case.
**Figure 6**: RMSEP (Root Mean Squared Error of Prediction) evolution as a function of the number of latent variables in the PLSR model when considering a reduced subset of 14 genes.
**Figure 7:** Detailed view of the PRESS evolution showed in Figure 5. Focus on four and five latent variables. *Num-*

*Genes* refers to the number of genes added to the predictive model apart from the initialization gene (SNORA38B).

**Figure 8:** Predicted versus actual subject fitness level obtained from the predictive model constructed with the top eight genes from Table 3 and adjusted by gender. Model applied to the basal gene expression profiles from 16 subject samples. '2' codes for elite and '1' codes for active subject. A hypothetical reference value or cut-off of 1.5 is included in the graph.

**Figure 9:** Boxplots of the log2 basal expression levels of the eight genes composing the optimum predictive model considering the subject fitness level.

**Figure 10:** Boxplots of the log2 basal expression levels of the further seven genes with prediction capabilities considering the subject fitness level.

**Figure 11:** Shows correlation with PLSR of individual fitness level (IF) calculated with eight genes in relation with race time (in hours) of subjects in a validation cohort. Model values are AdjR2=0.2037; Intercept = 4.23; Slope = -0.4374; SE_slope=0.1733; and pval_slope=0.02017.

**Figure 12:** Shows correlation with PLSR of individual fitness level (IF) calculated with fourteen genes in relation with race time (in hours) of subjects in a validation cohort. Model values are AdjR2=0.1952; Intercept = -2.744; Slope = -0.3215; SE_slope=0.1302; and pval_slope=0.0227.

## DETAILED DESCRIPTION OF THE INVENTION

*First method for determining the fitness of a subject*

In a first aspect the invention relates to an *in vitro* method for determining the fitness of a subject that comprises determining the expression level of at least one gene selected from the group consisting of GZMK, SNORA38B, and IGF2R in a sample from said subject, said sample selected from blood, blood plasma and serum.

In a particular embodiment the *in vitro* comprises determining the expression level of GZMK, SNORA38B, and IGF2R.

In another particular embodiment, the *in vitro* method for determining the fitness of a subject comprises determining the expression level of the genes GZMK, SNORA38B, IGF2R, LILRA6, KLF7, SPATA2, RAB19, and ATP6V0D1 in a sample from said subject, said sample selected from blood, blood plasma and serum.

More in particular, the *in vitro* method for determining the fitness of a subject according to this first aspect comprises:

(i) determining the expression level of the genes GZMK, SNORA38B, IGF2R, LILRA6, KLF7, SPATA2, RAB19, and ATP6V0D1 in a sample from said subject, said sample selected from blood, blood plasma and serum; and

(ii) calculating a predictive factor based on the expression levels of said gene(s), wherein said expression levels are corrected by a particular coefficient for each of the gene(s),

wherein said predictive factor is indicative of the fitness of the subject, and wherein the coefficient for each of the genes is a coefficient as shown in column 4 of Table 3 in the examples.

In another particular embodiment the *in vitro* method according to the first aspect, further comprises determining the expression levels of one or more genes selected from the group consisting of SPRY3, GPR97, SNORD116-24, SLC43A2, ZSWIM6, and LOC399715 in a sample from said subject, selected from blood, blood plasma and serum.

In another particular embodiment a predictive factor is calculated based on the expression levels of GZMK, SNORA38B, IGF2R, LILRA6, KLF7, SPATA2, RAB19, ATP6V0D1, SPRY3, GPR97, SNORD116-24, SLC43A2, ZSWIM6, and LOC399715 genes, and wherein said expression levels are corrected by a particular coefficient as shown in column 5 of Table 3 for each of the genes.

This description discloses an *in vitro* method for determining the fitness of a subject, hereinafter first method of the invention, that comprises:

(i) determining the expression level of at least one gene selected from the group consisting of GZMK, SNORA38B, IGF2R, LILRA6, KLF7, SPATA2, RAB19, and ATP6V0D1 in a sample from said subject, and

(ii) calculating a predictive factor based on the expression levels of said genes, wherein said expression levels are corrected by a particular coefficient for each of the genes,

wherein said predictive factor is indicative of the fitness of the subject.

The term *"in vitro"*, as used herein, refers to the fact that the method is not carried out on the body of a human or animal subject, but rather on cells or fluids isolated from said subject or in a test tube.

The term "subject" relates to human beings, including a male or female human being of any age or race. In a particular embodiment, the subject is an athlete. The term "athlete", as used herein, refers to a person who practices one or more sports that involve physical strength, speed or endurance. The term "athlete" includes both professional and amateur athletes. In a particular embodiment the athlete practices and endurance sport, preferably an endurance sport selected from the group consisting of endurance or long-distance running, swimming, cycling, skiing and race walking. The term "endurance sport" is defined in the context of the third aspect of the invention. In a more particular embodiment, the athlete is a runner. The term "runner", as used herein, refers to a person who runs for sport or pleasure, including long-distance or endurance running, middle-distance running and sprints. In an even more particular embodiment, the subject is an endurance runner. The term "endurance running" or "long-distance running", as used herein, refers to running over distances of at least three kilometers, including 5000 meters, 10000 meters, half-marathon (21,0975 km), marathon (42,195 km), ultramarathon (more than 42,195 km), trail (up to 42 km) and ultra-trail (more than 42 km). In an even more particular embodiment, the endurance runner is an ultra-trail runner. The term "ultra-trail runner", as used herein, refers to a runner of a trail race, i.e., a race over trails, of more than 42 km, including medium ultra-trail (from 42 to 69 km), long ultra-trail (from 70 to 99 km) and extra-long ultra-trail (100 km or more).

The term "fitness" or "fitness level", as used herein, refers to a general state of good physical condition and ability to perform aspects of sports, occupations and daily activities, usually resulting from physical exercise, proper nutrition and sufficient rest. Subjects can be classified in different categories according to their fitness.

Fitness can be achieved by practicing physical exercise, and the fitness of a subject is therefore associated with the amount of physical exercise the subject practices. The physical exercise can be aerobic, anaerobic or both. "Aerobic exercise" is understood as a physical exercise performed at a moderate level of intensity over a relatively long period of time. During aerobic exercise, heart and respiratory rates increase but the exercise can be maintained for an extended period of time. Illustrative non-limitative examples of aerobic exercises are running, jogging, elliptical training, walking, treadmill training, swimming, cycling, skinning, skipping rope, stair climbing, etc.). "Anaerobic exercise" is understood as a physical exercise performed at high intensity during a short period of time. It is used by athletes in non-endurance sports to promote strength, speed and power. Illustrative non-limitative examples of anaerobic exercise are weight lifting, sprinting and high-intensity interval training.

In a particular embodiment, the fitness of a subject can be elite or professional, active or acceptable and passive or sedentary.

The term "elite" or "professional", as used herein, refers to a subject with a very good level of fitness. In a particular embodiment, an elite subject practice more than 10 hours a week of physical exercise.

The term "active" or "acceptable", as used herein, refers to a subject with a regular to good level of fitness. In a particular embodiment, an active subject practices between three and ten hours a week of physical exercise.

The term "passive" or "sedentary", as used herein, refers to a subject with a poor level of fitness. In a particular embodiment, a sedentary subject practices less than three hours of physical exercise a week.

The first method of the invention comprises determining the expression level of at least one gene selected from the group consisting of GZMK, SNORA38B, and IGF2R, and in a particular embodiment additionally one of LILRA6, KLF7, SPATA2, RAB19 and ATP6V0D1 in a sample from the subject, said sample selected from blood, blood plasma and serum.

The term "expression level", as used herein, refers to a measurable quantity of a gene product produced by the gene in a sample of the subject, wherein the gene product can be a transcriptional product or a translational product. As understood by the person skilled in the art, the gene expression level can be quantified by measuring the levels of the transcriptional product of said gene (messenger RNA when the gene is a protein-coding gene) or of the protein encoded by said gene.

The level of a messenger RNA can be determined by methods well known in the art. For example the nucleic acid contained in the sample is first extracted according to standard methods, for example using lytic enzymes or chemical solutions or extracted by nucleic-acid-binding resins following the manufacturer's instructions. The extracted mRNA is then detected by hybridization (e.g., Northern blot analysis or by oligonucleotide microarrays after converting the mRNA into a labeled cDNA) and/or amplification (e.g., RT-PCR). Quantitative or semi-quantitative RT-PCR is preferred. Real-time quantitative or semiquantitative RT-PCR is particularly advantageous. Preferably, primer pairs are designed in order to overlap an intron, so as to distinguish cDNA amplification from putative genomic contamination. Suitable primers may be easily designed by the skilled person. Other methods of amplification include ligase chain reaction (LCR), transcription-mediated amplification (TMA), strand displacement amplification (SDA) and nucleic acid sequence based amplification (NASBA).

The level of a protein can be determined by any method known in the art suitable for the determination and quantification of a protein in a sample. By way of a non-limiting illustration, the level of a protein can be determined by means of a technique which comprises the use of antibodies with the capacity for binding specifically to the assayed protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. The antibodies can be monoclonal, polyclonal or fragments thereof, Fv, Fab, Fab' and F(ab')2, scFv, diabodies, triabodies, tetrabodies and humanized antibodies. Similarly, the antibodies may be labeled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. There is a wide variety of known tests that can be used according to the present invention, such as combined application of non-labeled antibodies (primary antibodies) and labeled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins include, for instance, affinity chromatography techniques or ligand-binding assays.

In a particular embodiment, the expression level of the at least one gene is determined by measuring the levels of the transcriptional product of said gene (messenger RNA when the gene is a protein-coding gene). In a more particular embodiment, the expression level of the at least one gene is determined by RT-PCR. In another particular embodiment, the expression level of the genes is determined by an oligonucleotide microarray.

The term "GZMK" or "TRYP2", as used herein, refers to a gene encoding the protein granzyme K. The human GZMK is assigned the Gene ID 3003 (NCBI GenBank, 7 May 2017 update). The term "GZMK" includes any of the transcript variants that have been described for this gene. The protein encoded by GZMK has the amino acid sequence defined under the accession number P49863 in UniProtKB/Swiss-Prot (version 142 of the entry as of 12 April 2017 and version 1 of the sequence as 1 October 1996).

The term "SNORA38B" or "small nucleolar RNA, H/ACA box 38B", as used herein, refers to a small nucleolar RNA of the H/ACA box family. The human SNORA38B is assigned the Gene ID 100124536 (NCBI GenBank, 3 April 2017 update). The term "SNORA38B" includes any of the transcript variants that have been described for this gene.

The term "IGF2R" or "MPRI", as used herein, refers to a gene encoding the protein cation-independent mannose-6-phosphate receptor (also known as insulin-like growth factor II receptor or M6P/IGF2 receptor). The human IGF2R is assigned the Gene ID 3482 (NCBI GenBank, 11 May 2017 update). The term "IGF2R" includes any of the transcript variants that have been described for this gene. The protein encoded by IGF2R has the amino acid sequence defined under the accession number P11717 in UniProtKB/Swiss-Prot (version 194 of the entry as of 10 May 2017 and version 3 of the sequence as 11 January 2011).

The term "LILRA6" or "ILT8", as used herein, refers to a gene encoding the protein "Leukocyte immunoglobulin-like receptor subfamily A member 6" (also known as "immunoglobulin-like transcript 8" or "leukocyte Ig-like receptor"). The human LILRA6 is assigned the Gene ID 79168 (NCBI GenBank, 15 May 2017 update). The term "LILRA6" includes any of the transcript variants that have been described for this gene. The protein encoded by LILRA6 has the amino acid sequence defined under the accession number Q6PI73 in UniProtKB/Swiss-Prot (version 110 of the entry as of 10 May 2017 and version 2 of the sequence as 10 July 2007).

The term "KLF7" or "TRYP2", as used herein, refers to a gene encoding the protein "Krueppel-like factor 7" (also known as "ubiquitous krueppel-like factor"). The human KLF7 is assigned the Gene ID 8609 (NCBI GenBank, 8 May 2017 update). The term "KLF7" includes any of the transcript variants that have been described for this gene. The protein encoded by KLF7 has the amino acid sequence defined under the accession number P49863 in UniProtKB/Swiss-Prot (version 142 of the entry as of 12 April 2017 and version 1 of the sequence as 1 October 1996).

The term "SPATA2" or "KIAA0757" or "PD1", as used herein, refers to a gene encoding the protein "spermatogenesis-associated protein 2" (also known as "spermatogenesis-associated protein PD1"). The human SPATA2 is assigned the Gene ID 9825 (NCBI GenBank, 7 May 2017 update). The term "SPATA2" includes any of the transcript variants that have been described for this gene. The protein encoded by SPATA2 has the amino acid sequence defined under the accession number Q9UM82 in UniProtKB/Swiss-Prot (version 137 of the entry as of 15 March 2017 and version 2 of the sequence as 27 April 2001).

The term "RAB19" or "RAB19B", as used herein, refers to a gene encoding the protein "Ras-related protein Rab-19". The human RAB19 is assigned the Gene ID 401409 (NCBI GenBank, 22 April 2017 update). The term "RAB19" includes any of the transcript variants that have been described for this gene. The protein encoded by RAB19 has the amino acid sequence defined under the accession number A4D1S5 in UniProtKB/Swiss-Prot (version 95 of the entry as of 10 May 2017 and version 2 of the sequence as 11 September 2007).

The term "ATP6V0D1" or "ATP6D" or "VPATPD", as used herein, refers to a gene encoding the protein "V-type proton ATPase subunit d 1" (also known as "32 kDa accessory protein" or "V-ATPase 40 kDa accessory protein" or "V-ATPase AC39 subunit" or "Vacuolar proton pump subunit d 1"). The human ATP6V0D1 is assigned the Gene ID 9114 (NCBI GenBank, 8 May 2017 update). The term "ATP6V0D1" includes any of the transcript variants that have been described for this gene. The protein encoded by ATP6V0D1 has the amino acid sequence defined under the accession number P61421 in UniProtKB/Swiss-Prot (version 129 of the entry as of 10 May 2017 and version 1 of the sequence as 24 May 2004).

In a particular embodiment, the first method of the invention comprises determining the expression level of only one gene selected from GZMK, SNORA38B, and IGF2R. In a preferred embodiment, the gene is GZMK.

In another particular embodiment, the first method of the invention comprises determining the expression level of two genes selected from GZMK, SNORA38B, and IGF2R, In another particular embodiment, the first method of the invention comprises determining the expression level of two genes selected from GZMK, SNORA38B, IGF2R, LILRA6, KLF7, SPATA2, RAB19, and ATP6V0D1, wherein one of the genes is GZMK, SNORA38B, or IGF2R. In another particular embodiment, the first method of the invention comprises determining the expression level of two genes selected from GZMK, SNORA38B IGF2R, LILRA6, KLF7, SPATA2, RAB19, and ATP6V0D1. In a preferred embodiment, the first method of the invention comprises determining the expression level of SNORA38B and one more gene selected from GZMK, IGF2R, LILRA6, KLF7, SPATA2, RAB19, and ATP6V0D1. In a more particular embodiment, the first method of the invention comprises determining the expression level of GZMK and SNORA38B, or GZMK and IGF2R, or GZMK and LILRA6, or GZMK and KLF7, or GZMK and SPATA2, or GZMK and RAB19, or GZMK and ATP6V0D1, or SNORA38B and IGF2R, or SNORA38B and LILRA6, or SNORA38B and KLF7, or SNORA38B and SPATA2, or SNORA38B and RAB19, or SNORA38B and ATP6V0D1, or IGF2R and LILRA6, or IGF2R and KLF7, or IGF2R and SPATA2, or IGF2R and RAB19, or IGF2R and ATP6V0D1. Other possible combinations of two genes are LILRA6 and KLF7, or LILRA6 and SPATA2, or LILRA6 and RAB19, or LILRA6 and ATP6V0D1, or KLF7 and SPATA2, or KLF7 and RAB19, or KLF7 and ATP6V0D1, or SPATA2 and RAB19, or SPATA2 and ATP6V0D1.

In another particular embodiment, the first method of the invention comprises determining the expression level of three genes selected from GZMK, SNORA38B, IGF2R, LILRA6, KLF7, SPATA2, RAB19, and ATP6V0D1. In another particular embodiment, the first method of the invention comprises determining the expression level of three genes selected from GZMK, SNORA38B, IGF2R, LILRA6, KLF7, SPATA2, RAB19, and ATP6V0D1, wherein one of the genes is at least GZMK, SNORA38B, or IGF2R. In a preferred embodiment, the first method of the invention comprises determining the expression level of SNORA38B and two more genes selected from GZMK, IGF2R, LILRA6, KLF7, SPATA2, RAB19, and ATP6V0D1. In a more particular embodiment, the first method of the invention comprises determining the expression level of GZMK, SNORA38B and IGF2R, or GZMK, SNORA38B and LILRA6, or GZMK, SNORA38B and KLF7, or GZMK, SNORA38B and SPATA2, or GZMK, SNORA38B and RAB19, or GZMK, SNORA38B, and ATP6V0D1, or GZMK, IGF2R, LILRA6, or GZMK, IGF2R and KLF7, or GZMK, IGF2R and SPATA2, or GZMK, IGF2R and RAB19, or GZMK, IGF2R and ATP6V0D1, or GZMK, LILRA6 and KLF7, or GZMK, LILRA6 and SPATA2, or GZMK, LILRA6 and RAB19, or GZMK, LILRA6 and ATP6V0D1, or GZMK, KLF7 and SPATA2, or GZMK, KLF7 and RAB19, or GZMK, KLF7 and ATP6V0D1, or GZMK, SPATA2 and RAB19, or GZMK, SPATA2 and ATP6V0D1, or GZMK, RAB19 and ATP6V0D1, or SNORA38B, IGF2R and LILRA6, or SNORA38B, IGF2R and KLF7 or SNORA38B, IGF2R and SPATA2, or SNORA38B, IGF2R and RAB19, or SNORA38B, IGF2R and ATP6V0D1, or SNORA38B, LILRA6 and KLF7, or SNORA38B, LILRA6 and SPATA2, or SNORA38B, LILRA6 and RAB19, or SNORA38B, LILRA6, and ATP6V0D1, or SNORA38B, KLF7 and SPATA2, or SNORA38B, KLF7 and RAB19, or SNORA38B, KLF7 and ATP6V0D1, or SNORA38B, SPATA2 and RAB19, or SNORA38B, SPATA2 and ATP6V0D1, or SNORA38B, RAB19 and ATP6V0D1, or IGF2R, LILRA6 and KLF7, or IGF2R, LILRA6 and SPATA2, or IGF2R, LILRA6 and RAB19, or IGF2R, LILRA6 and ATP6V0D1, or IGF2R, KLF7 and SPATA2, or IGF2R, KLF7 and RAB19, or IGF2R, KLF7 and ATP6V0D1, or IGF2R, SPATA2 and RAB19, or IGF2R, SPATA2 and ATP6V0D1, or IGF2R, RAB19 and ATP6V0D1. Other possible combinations of three genes exemplified in this description are LILRA6, KLF7 and SPATA2, or LILRA6, KLF7 and RAB19, or LILRA6, KLF7 and ATP6V0D1, or LILRA6, SPATA2 and RAB19, or LILRA6, SPATA2 and ATP6V0D1, or LILRA6, RAB19 and ATP6V0D1, or KLF7, SPATA2 and RAB19, or KLF7, SPATA2 and ATP6V0D1, or KLF7, RAB19 and ATP6V0D1, or SPATA2, RAB19 and ATP6V0D1. This description discloses the expression level of three genes selected from GZMK, SNORA38B, IGF2R, LILRA6, KLF7, SPATA2, RAB19, and ATP6V0D1 for determining the fitness of a subject.

In another particular embodiment, the first method of the invention comprises determining the expression level of four genes selected from GZMK, SNORA38B, IGF2R, LILRA6, KLF7, SPATA2, RAB19, and ATP6V0D1. In another particular embodiment, the first method of the invention comprises determining the expression level of four genes selected from GZMK, SNORA38B, IGF2R, LILRA6, KLF7, SPATA2, RAB19, and ATP6V0D1, wherein one of the genes is at least GZMK, SNORA38B, or IGF2R. In a preferred embodiment, the first method of the invention comprises determining the expression level of SNORA38B and three more genes selected from GZMK, IGF2R, LILRA6, KLF7, SPATA2, RAB19, and ATP6V0D1. In a more particular embodiment, the first method of the invention comprises determining the expression level of GZMK, SNORA38B, IGF2R and LILRA6, or GZMK, SNORA38B, IGF2R and KLF7, or GZMK, SNORA38B, IGF2R and SPATA2, or GZMK, SNORA38B, IGF2R and RAB19, or GZMK, SNORA38B, IGF2R and ATP6V0D1, or GZMK, SNORA38B, LILRA6 and KLF7, or GZMK, SNORA38B, LILRA6 and SPATA2, or GZMK, SNORA38B, LILRA6 and RAB19, or GZMK, SNORA38B, LILRA6 and ATP6V0D1, or GZMK, SNORA38B, KLF7 and SPATA2, or GZMK, SNORA38B, KLF7 and RAB19, or GZMK, SNORA38B, KLF7 and ATP6V0D1, or GZMK, SNORA38B, SPATA2 and RAB19, or GZMK, SNORA38B, SPATA2 and ATP6V0D1, or GZMK, SNORA38B, RAB19 and ATP6V0D1, or GZMK, IGF2R, LILRA6 and KLF7, or GZMK, IGF2R, LILRA6 and SPATA2, or GZMK, IGF2R, LILRA6 and RAB19, or GZMK, IGF2R, LILRA6 and ATP6V0D1, or GZMK, IGF2R, KLF7 and SPATA2, or GZMK, IGF2R, KLF7 and RAB19, or GZMK, IGF2R, KLF7 and ATP6V0D1, or GZMK, IGF2R, SPATA2 and RAB19, or GZMK, IGF2R, SPATA2 and ATP6V0D1, or GZMK, IGF2R, RAB19 and ATP6V0D1, or GZMK, LILRA6, KLF7 and SPATA2, or GZMK, LILRA6, KLF7 and RAB19, or GZMK, LILRA6, KLF7 and ATP6V0D1, or GZMK, LILRA6, SPATA2 and RAB19, or GZMK, LILRA6, SPATA2 and ATP6V0D1, or GZMK, LILRA6, RAB19 and ATP6V0D1, or GZMK, KLF7, SPATA2 and RAB19, or GZMK, KLF7, SPATA2 and ATP6V0D1, or GZMK, KLF7, RAB19 and ATP6V0D1, or GZMK, SPATA2, RAB19 and ATP6V0D1, or SNORA38B, IGF2R, LILRA6 and KLF7, or SNORA38B, IGF2R, LILRA6 and SPATA2, or SNORA38B, IGF2R, LILRA6 and RAB19, or SNORA38B, IGF2R, LILRA6 and ATP6V0D1, or SNORA38B, IGF2R, KLF7 and SPATA2, or SNORA38B, IGF2R, KLF7 and RAB19, or SNORA38B, IGF2R, KLF7 and ATP6V0D1, or SNORA38B, IGF2R, SPATA2 and RAB19, or SNORA38B, IGF2R, SPATA2 and ATP6V0D1, or SNORA38B, IGF2R, RAB19 and ATP6V0D1, or SNORA38B, LILRA6, KLF7 and SPATA2, or SNORA38B, LILRA6, KLF7 and RAB19, or SNORA38B, LILRA6, KLF7 and ATP6V0D1, or SNORA38B, LILRA6, SPATA2 and RAB19, or SNORA38B, LILRA6, SPATA2 and ATP6V0D1, or SNORA38B, LILRA6, RAB19 and ATP6V0D1, or SNORA38B, KLF7, SPATA2 and RAB19, or SNORA38B, KLF7, SPATA2 and ATP6V0D1, or SNORA38B, KLF7, RAB19 and ATP6V0D1, or SNORA38B, SPATA2, RAB19 and ATP6V0D1, or IGF2R, LILRA6, KLF7 and SPATA2, or IGF2R, LILRA6, KLF7 and RAB19, or IGF2R, LILRA6, KLF7 and ATP6V0D1, or IGF2R, LILRA6, SPATA2 and RAB19, or IGF2R, LILRA6, SPATA2 and ATP6V0D1, or IGF2R, LILRA6, RAB19 and ATP6V0D1, or IGF2R, KLF7, SPATA2 and RAB19, or IGF2R, KLF7, SPATA2 and ATP6V0D1, or IGF2R, KLF7, RAB19 and ATP6V0D1, or IGF2R, SPATA2, RAB19 and ATP6V0D1. Other possible combinations of four genes exemplified in this description are LILRA6, KLF7, SPATA2 and RAB19, or LILRA6, KLF7, SPATA2 and ATP6V0D1, or LILRA6, KLF7, RAB19 and ATP6V0D1, or LILRA6, SPATA2, RAB19 and ATP6V0D1, or KLF7, SPATA2, RAB19 and ATP6V0D1. This description discloses the expression level of four genes selected from GZMK, SNORA38B, IGF2R, LILRA6, KLF7, SPATA2, RAB19, and ATP6V0D1 for determining the fitness of a subject.

In another particular embodiment, the first method of the invention comprises determining the expression level of five genes selected from GZMK, SNORA38B, IGF2R, LILRA6, KLF7, SPATA2, RAB19, and ATP6V0D1. In another particular embodiment, the first method of the invention comprises determining the expression level of five genes selected from GZMK, SNORA38B, IGF2R, LILRA6, KLF7, SPATA2, RAB19, and ATP6V0D1, wherein at least one gene is GZMK, SNORA38B, or IGF2R. In a preferred embodiment, the first method of the invention comprises determining the expression level of SNORA38B and four more genes selected from GZMK, IGF2R, LILRA6, KLF7, SPATA2, RAB19, and ATP6V0D1. In a more particular embodiment, the first method of the invention comprises determining the expression level of GZMK, SNORA38B, IGF2R, LILRA6 and KLF7, or GZMK, SNORA38B, IGF2R, LILRA6 and SPATA2, or GZMK, SNORA38B, IGF2R, LILRA6 and RAB19, or GZMK, SNORA38B, IGF2R, LILRA6 and ATP6V0D1, or GZMK, SNORA38B, IGF2R, KLF7 and SPATA2, or GZMK, SNORA38B, IGF2R, KLF7 and RAB19, or GZMK, SNORA38B, IGF2R, KLF7 and ATP6V0D1, or GZMK, SNORA38B, IGF2R, SPATA2 and RAB19, or GZMK, SNORA38B, IGF2R, SPATA2 and ATP6V0D1, or GZMK, SNORA38B, IGF2R, RAB19 and ATP6V0D1, or GZMK, SNORA38B, LILRA6, KLF7 and SPATA2, or GZMK, SNORA38B, LILRA6, KLF7 and RAB19, or GZMK, SNORA38B, LILRA6, KLF7 and ATP6V0D1, or GZMK, SNORA38B, LILRA6, SPATA2 and RAB19, or GZMK, SNORA38B, LILRA6, SPATA2 and ATP6V0D1, or GZMK, SNORA38B, LILRA6, RAB19 and ATP6V0D1, or GZMK, SNORA38B, KLF7, SPATA2 and RAB19, or GZMK, SNORA38B, KLF7, SPATA2 and ATP6V0D1, or GZMK, SNORA38B, KLF7, RAB19 and ATP6V0D1, or GZMK, SNORA38B, SPATA2, RAB19 and ATP6V0D1, or GZMK, IGF2R, LILRA6, KLF7 and SPATA2, or GZMK, IGF2R, LILRA6, KLF7 and RAB19, or GZMK, IGF2R, LILRA6, KLF, and ATP6V0D1, or GZMK, IGF2R, LILRA6, SPATA2 and RAB19, or GZMK, IGF2R, LILRA6, SPATA2 and ATP6V0D1, or GZMK, IGF2R, LILRA6, RAB19 and ATP6V0D1, or GZMK, IGF2R, KLF7, SPATA2 and RAB19, or GZMK, IGF2R, KLF7, SPATA2 and ATP6V0D1, or GZMK, IGF2R, KLF7, RAB19 and ATP6V0D1, or GZMK, IGF2R, SPATA2, RAB19 and ATP6V0D1, or GZMK, LILRA6, KLF7, SPATA2 and RAB19, or GZMK, LILRA6, KLF7, SPATA2 and ATP6V0D1, or GZMK, LILRA6, KLF7, RAB19 and ATP6V0D1, or GZMK, LILRA6, SPATA2, RAB19 and ATP6V0D1, or GZMK, KLF7, SPATA2, RAB19 and ATP6V0D1, or SNORA38B, IGF2R, LILRA6, KLF7 and SPATA2, or SNORA38B, IGF2R, LILRA6, KLF7 and RAB19, or SNORA38B, IGF2R, LILRA6, KLF7 and ATP6V0D1, or SNORA38B, IGF2R, LILRA6, SPATA2 and RAB19, or SNORA38B, IGF2R, LILRA6, SPATA2 and ATP6V0D1, or SNORA38B, IGF2R, LILRA6, RAB19 and ATP6V0D1, or SNORA38B, IGF2R, KLF7, SPATA2 and RAB19, or SNORA38B, IGF2R, KLF7, SPATA2 and ATP6V0D1, or SNORA38B, IGF2R, KLF7, RAB19 and ATP6V0D1, or SNORA38B, IGF2R, SPATA2, RAB19 and ATP6V0D1, or SNORA38B, LILRA6, KLF7, SPATA2 and RAB19, or SNORA38B, LILRA6, KLF7, SPATA2 and ATP6V0D1, or SNORA38B, LILRA6, KLF7, RAB19 and ATP6V0D1, or SNORA38B, LILRA6, SPATA2, RAB19 and ATP6V0D1, or SNORA38B, KLF7, SPATA2, RAB19 and ATP6V0D1, or IGF2R, LILRA6, KLF7, SPATA2 and RAB19, or IGF2R, LILRA6, KLF7, SPATA2 and ATP6V0D1, or IGF2R, LILRA6, KLF7, RAB19 and ATP6V0D1, or IGF2R, LILRA6, SPATA2, RAB19 and ATP6V0D1, or IGF2R, KLF7, SPATA2, RAB19 and ATP6V0D1. Other possible combinations of four genes exemplified in this description are LILRA6, KLF7, SPATA2, RAB19 and ATP6V0D1.

In another particular embodiment, the first method of the invention comprises determining the expression level of six genes selected from GZMK, SNORA38B, IGF2R, LILRA6, KLF7, SPATA2, RAB19, and ATP6V0D1. In another particular embodiment, the first method of the invention comprises determining the expression level of six genes selected from GZMK, SNORA38B, IGF2R, LILRA6, KLF7, SPATA2, RAB19, and ATP6V0D1, wherein at least one gene is GZMK, SNORA38B, or IGF2R. In a preferred embodiment, the first method of the invention comprises determining the expression level of SNORA38B and five more genes selected from GZMK, IGF2R, LILRA6, KLF7, SPATA2, RAB19, and ATP6V0D1. In a more particular embodiment, the first method of the invention comprises determining the expression level of GZMK, SNORA38B, IGF2R, LILRA6, KLF7 and SPATA2, or GZMK, SNORA38B, IGF2R, LILRA6, KLF7 and RAB19, or GZMK, SNORA38B, IGF2R, LILRA6, KLF7 and ATP6V0D1, or GZMK, SNORA38B, IGF2R, LILRA6, SPATA2 and RAB19, or GZMK, SNORA38B, IGF2R, LILRA6, SPATA2 and ATP6V0D1, or GZMK, SNORA38B, IGF2R, LILRA6, RAB19 and ATP6V0D1, or GZMK, SNORA38B, IGF2R, KLF7, SPATA2 and RAB19, or GZMK, SNORA38B, IGF2R, KLF7, SPATA2 and ATP6V0D1, or GZMK, SNORA38B, IGF2R, KLF7, RAB19 and ATP6V0D1, or GZMK, SNORA38B, IGF2R, SPATA2, RAB19 and ATP6V0D1, or GZMK, SNORA38B, LILRA6, KLF7, SPATA2 and RAB19, or GZMK, SNORA38B, LILRA6, KLF7, SPATA2 and ATP6V0D1, or GZMK, SNORA38B, LILRA6, KLF7, RAB19 and ATP6V0D1, or GZMK, SNORA38B, LILRA6, SPATA2, RAB19 and ATP6V0D1, or GZMK, SNORA38B, KLF7, SPATA2, RAB19 and ATP6V0D1, or GZMK, IGF2R, LILRA6, KLF7, SPATA2 and RAB19, or GZMK, IGF2R, LILRA6, KLF7, SPATA2 and ATP6V0D1, or GZMK, IGF2R, LILRA6, KLF7, RAB19 and ATP6V0D1, or GZMK, IGF2R, LILRA6, SPATA2, RAB19 and ATP6V0D1, or GZMK, IGF2R, KLF7, SPATA2, RAB19 and ATP6V0D1, or GZMK, LILRA6, KLF7, SPATA2, RAB19 and ATP6V0D1, or SNORA38B, IGF2R, LILRA6, KLF7, SPATA2 and RAB19, or SNORA38B, IGF2R, LILRA6, KLF7, SPATA2 and ATP6V0D1, or SNORA38B, IGF2R, LILRA6, KLF7, RAB19 and ATP6V0D1, or SNORA38B, IGF2R, LILRA6, SPATA2, RAB19 and ATP6V0D1, or SNORA38B, IGF2R, KLF7, SPATA2, RAB19 and ATP6V0D1, or SNORA38B, LILRA6, KLF7, SPATA2, RAB19 and ATP6V0D1, or IGF2R, LILRA6, KLF7, SPATA2, RAB19 and ATP6V0D1.

In another particular embodiment, the first method of the invention comprises determining the expression level of seven genes selected from GZMK, SNORA38B, IGF2R, LILRA6, KLF7, SPATA2, RAB19, and ATP6V0D1. In another particular embodiment, the first method of the invention comprises determining the expression level of seven genes selected from GZMK, SNORA38B, IGF2R, LILRA6, KLF7, SPATA2, RAB19, and ATP6V0D1, wherein at least one gene is GZMK, SNORA38B, or IGF2R. In a preferred embodiment, the first method of the invention comprises determining the expression level of SNORA38B and six more genes selected from GZMK, IGF2R, LILRA6, KLF7, SPATA2, RAB19, and ATP6V0D1. In a more particular embodiment, the first method of the invention comprises determining the expression level of GZMK, SNORA38B, IGF2R, LILRA6, KLF7, SPATA2 and RAB19, or GZMK, SNORA38B, IGF2R, LILRA6, KLF7, SPATA2 and ATP6V0D1, or GZMK, SNORA38B, IGF2R, LILRA6, KLF7, RAB19 and ATP6V0D1, or GZMK, SNORA38B, IGF2R, LILRA6, SPATA2, RAB19 and ATP6V0D1, or GZMK, SNORA38B, IGF2R, KLF7, SPATA2, RAB19 and ATP6V0D1, or GZMK, SNORA38B, LILRA6, KLF7, SPATA2, RAB19 and ATP6V0D1, or GZMK, IGF2R, LILRA6, KLF7, SPATA2, RAB19 and ATP6V0D1, or SNORA38B, IGF2R, LILRA6, KLF7, SPATA2, RAB19 and ATP6V0D1.

As above indicated, in another particular embodiment, the first method of the invention comprises determining the expression level of GZMK, SNORA38B, IGF2R, LILRA6, KLF7, SPATA2, RAB19 and ATP6V0D1.

In a particular embodiment, the first method of the invention further comprises determining the expression levels of one or more genes selected from the group consisting of SPRY3, GPR97, SNORD116-24, SLC43A2, ZSWIM6 and LOC399715.

The term "SPRY3", as used herein, refers to a gene encoding the protein "protein sprout homolog 3". The human SPRY3 is assigned the Gene ID 10251 (NCBI GenBank, 8 May 2017 update). The term "SPRY3" includes any of the transcript variants that have been described for this gene. The protein encoded by ATP6V0D1 has the amino acid sequence defined under the accession number 043610 in UniProtKB/Swiss-Prot (version 124 of the entry as of 15 March 2017 and version 2 of the sequence as 21 February 2001).

The term "GPR97" or "ADGRG3" or "PGR26", as used herein, refers to a gene encoding the protein "Adhesion G protein-coupled receptor G3" (also known as "G-protein coupled receptor 97" or "G-protein coupled receptor PGR26"). The human GPR97 is assigned the Gene ID 222487 (NCBI GenBank, 22 April 2017 update). The term "GPR97" includes any of the transcript variants that have been described for this gene. The protein encoded by GPR97 has the amino acid sequence defined under the accession number Q86Y34 in UniProtKB/Swiss-Prot (version 124 of the entry as of 10 May 2017 and version 1 of the sequence as 1 June 2003).

The term "SNORD116-24" or "small nucleolar RNA, C/D box 116-24", as used herein, refers to a small nucleolar RNA of the family C/D box. The human ATP6V0D1 is assigned the Gene ID 100033435 (NCBI GenBank, 8 May 2017 update). The term "SNORD116-24" includes any of the transcript variants that have been described for this gene.

The term "SLC43A2" or "LAT4", as used herein, refers to a gene encoding the protein "V Large neutral amino acids transporter small subunit 4" (also known as "L-type amino acid transporter 4" or "Solute carrier family 43 member 2"). The human SLC43A2 is assigned the Gene ID 124935 (NCBI GenBank, 8 May 2017 update). The term "SLC43A2" includes any of the transcript variants that have been described for this gene. The protein encoded by SLC43A2 has the amino acid sequence defined under the accession number Q8N370 in UniProtKB/Swiss-Prot (version 120 of the entry as of 10 May 2017 and version 1 of the sequence as 1 October 2002).

The term "ZSWIM6" or "KIAA1577", as used herein, refers to a gene encoding the protein "Zinc finger SWIM domain-containing protein 6". The human ZSWIM6 is assigned the Gene ID 57688 (NCBI GenBank, 8 May 2017 update). The term "ZSWIM6" includes any of the transcript variants that have been described for this gene. The protein encoded by ZSWIM6 has the amino acid sequence defined under the accession number Q9HCJ5 in UniProtKB/Swiss-Prot (version 99 of the entry as of 15 March 2017 and version 2 of the sequence as 5 May 2009).

The term "LOC399715", as used herein, refers to a non-coding RNA. The human ATP6V0D1 is assigned the Gene ID 399715 (NCBI GenBank, 2 April 2017 update).

In a particular embodiment, the first method of the invention further comprises determining the expression level of at least one, at least two, at least three, at least four, at least five or the six genes selected from the group consisting of SPRY3, GPR97, SNORD116-24, SLC43A2, ZSWIM6 and LOC399715. In a more particular embodiment, the first method of the invention comprises determining the expression levels of SPRY3, GPR97, SNORD116-24, SLC43A2, ZSWIM6 and LOC399715.

In a particular embodiment, the first method of the invention comprises determining the expression level of at least one, at least two, at least three, at least four, at least five, at least six, at least seven or the eight genes selected from the group consisting of GZMK, SNORA38B, IGF2R, LILRA6, KLF7, SPATA2, RAB19 and ATP6V0D1 and at least one, at least two, at least three, at least four, at least five or the six genes selected from the group consisting of SPRY3, GPR97, SNORD116-24, SLC43A2, ZSWIM6 and LOC399715. In a more particular embodiment, the first method of the invention comprises determining the expression level of GZMK, SNORA38B, IGF2R, LILRA6, KLF7, SPATA2, RAB19, ATP6V0D1, SPRY3, GPR97, SNORD116-24, SLC43A2, ZSWIM6 and LOC399715.

The term "sample" or "biological sample", as used herein, refers to biological material isolated from a subject. The biological sample may contain any biological material suitable for detecting the desired biomarker and may comprise cellular and/or non-cellular material from the subject. The sample can be isolated from any suitable biological tissue or fluid such as, for example, blood, blood plasma, serum, urine, cerebral spinal fluid (CSF), saliva, sputum, deposition, tears, mucus, and sweat. In a particular embodiment, the samples are blood samples. In a more particular embodiment, the samples are venous blood samples.

The first method of the invention comprises calculating a predictive factor based on the expression levels of the above-mentioned genes corrected by a particular coefficient for each of the genes.

The term "predictive factor", as used herein, refers to a factor that is derived from the expression levels of the above-mentioned genes. The predictive factor can be calculated by summing the expression values obtained for each gene in the step (i) of the method corrected by a particular coefficient for each of the genes. Statistical methods for calculating such correction coefficients are known to those skilled in the art. In a particular embodiment, the correction coefficients are directly the weight values obtained from fitting a Partial Least Squares Regression (PLSR) with the above-mentioned gene expression levels as predictor variables together with the subject gender and an intercept for the adjusted model. This PLSR comprises a group of subjects from whom their fitness level is already known. This group of subjects can be referred as the training set for constructing the predictive model. The response variable in this PLSR is the subject fitness level coded with a numeric value. In this sense, the predictive factor is indicative of the fitness of the subject in the way it has been numerically coded in the training set. In a particular embodiment, as used in the examples of the present application, an elite/professional subject is coded with a numeric value higher than that used for coding the active/acceptable subject, for example, the elite/professional is coded with 2 and the active/acceptable is coded with 1. In this particular embodiment, a passive/sedentary subject should be coded with a numeric value lower than that used for coding the active/acceptable subject and the elite/professional subject. For example, if the elite/professional is coded with 2 and the active/acceptable is coded with 1, the passive/sedentary subject can be coded with number 0. In this particular embodiment, the predictive factor is proportional to the fitness level of the subject. In another particular embodiment, an elite/professional subject is coded with a numeric value lower that used for coding the active/acceptable subject, for example, a subject with elite or professional fitness level is coded with 1 and a subject with active or acceptable fitness level is coded with 2. In this particular embodiment, a passive/sedentary subject should be coded with a numeric value higher than that used for coding the active/acceptable subject and the elite/professional subject. For example, if the elite/professional is coded with 1 and the active/acceptable is coded with 2, the passive/sedentary subject can be coded with number 3. In this particular embodiment, the predictive factor is inversely proportional to the fitness level of the subject.

As indicated above, in an even more particular embodiment, the expression levels of the genes GZMK, SNORA38B, IGF2R, LILRA6, KLF7, SPATA2, RAB19, and ATP6V0D1 are determined and the correction coefficient for each of the genes is a coefficient as shown in column 4 of Table 3. In another particular embodiment, the expression levels of the genes GZMK, SNORA38B, IGF2R, LILRA6, KLF7, SPATA2, RAB19, ATP6V0D1, SPRY3, GPR97, SNORD116-24, SLC43A2, ZSWIM6 and LOC399715 are determined and the correction coefficient for each of the genes is a coefficient as shown in column 5 of Table 3.

In a more particular embodiment, when the first method of the invention comprises determining the expression levels of GZMK, SNORA38B, IGF2R, LILRA6, KLF7, SPATA2, RAB19, ATP6V0D1, the variable gender is corrected by a coefficient of 0.1652. In another particular embodiment, when the first method of the invention comprises determining the expression levels of GZMK, SNORA38B, IGF2R, LILRA6, KLF7, SPATA2, RAB19, ATP6V0D1, SPRY3, GPR97, SNORD116-24, SLC43A2, ZSWIM6 and LOC399715, the variable gender is corrected by a coefficient of 0.1389.

In a particular embodiment, for calculating the predictive factor the intercept is taken into consideration. In a more particular embodiment, when the first method of the invention comprises determining the expression levels of GZMK, SNORA38B, IGF2R, LILRA6, KLF7, SPATA2, RAB19, ATP6V0D1, the intercept value is -0.94103. In another particular embodiment, when the first method of the invention comprises determining the expression levels of GZMK, SNORA38B, IGF2R, LILRA6, KLF7, SPATA2, RAB19, ATP6V0D1, SPRY3, GPR97, SNORD116-24, SLC43A2, ZSWIM6 and LOC399715, the intercept value is -11.5154.

In an even more particular embodiment, the predictive factor is calculated using the following equation (1):

$$\text{Predictive Factor} \approx w_0 + w_1 \cdot gender + \sum_{i=2}^{n} w_i \cdot g_i \qquad (1)$$

where $w_0$ is the PLSR model intercept, $w_1$ and $w_i$ are the weights or correction coefficients for variables *gender* and basal gene expression levels of the above-mentioned genes $g_i$ respectively.

In a particular embodiment, the first method of the invention further comprises comparing the predictive factor calculated in (ii) with a reference value and classifying the subject according to their fitness as professional/elite, active/acceptable or passive/sedentary.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on a large number of samples, such as from population of subjects of the chronological age matched group, or based on a pool of samples.

The reference value according to the first method of the invention can be obtained from two or more subjects with two different known fitness levels, or from three or more subjects with three different known fitness levels, from whom the predictive factor is to be computed the same way as the predictive factor of the subject whose fitness is being tested. In a particular embodiment, the reference value is selected to maximize the area under a ROC curve (AUC). In another particular embodiment, as used herein, the reference value is selected so the classification error rate is minimized.

In a particular embodiment, the reference value is obtained from one or more elite or professional subjects and one or more active or acceptable subjects. In a particular embodiment, in case of coding an elite/professional subject with a numeric value lower than that used for the active/acceptable subject, if the predictive factor is lower than the reference value, the subject is considered elite/professional, and if the predictive factor is higher than the reference value, the subject is considered active/acceptable or passive/sedentary. In another particular embodiment, in case of coding an active/acceptable with a numeric value higher than that used for the passive/sedentary, if the predictive factor is higher than the reference value, the subject is considered active/acceptable, and if the predictive factor is lower than the reference value, the subject is considered passive/sedentary.

In a particular embodiment, the reference value is obtained from one or more elite or professional subjects and one or more passive or sedentary subjects. In a particular embodiment, in case of coding an elite/professional with a numeric value lower than that used for the passive/sedentary, if the predictive factor is lower than the reference value, the subject is considered elite/professional, and if the predictive factor is higher than the reference value, the subject is considered passive/sedentary. In another particular embodiment, in case of coding an elite/professional with a numeric value higher than that used for the passive/sedentary, if the predictive factor is higher than the reference value, the subject is considered elite/professional, and if the predictive factor is lower than the reference value, the subject is considered passive/sedentary.

In a particular embodiment, the reference value is obtained from one or more active or acceptable subjects and one or more passive or sedentary subjects. In a particular embodiment, in case of coding an active/acceptable with a numeric value lower than that used for the passive/sedentary, if the predictive factor is lower than the reference value, the subject is considered active/acceptable, and if the predictive factor is higher than the reference value, the subject is considered passive/sedentary. In another particular embodiment, in case of coding an active/acceptable with a numeric value higher than that used for the passive/sedentary, if the predictive factor is higher than the reference value, the subject is considered active/acceptable, and if the predictive factor is lower than the reference value, the subject is considered passive/sedentary.

In a particular embodiment, two references values are obtained from one or more elite/professional subjects, one or more active/acceptable subjects and one or more passive/sedentary subjects. First reference value differentiates between elite/professional subjects and active/acceptable or passive/sedentary subjects and second reference value differentiates between passive/sedentary subjects and elite/professional subjects or active/acceptable. In a particular embodiment, in case of coding a passive/sedentary subject with the lowest numeric value, if the predictive factor is (i) lower than the second reference value, the subject is considered passive/sedentary or (ii) higher than the first reference value, the subject is considered elite/professional or (iii) higher than the second reference value and lower than the first reference value, the subject is considered active/acceptable. In another particular embodiment, in case of coding a passive/sedentary subject with the highest numeric value, if the predictive factor is (i) higher than the second reference value, the subject is considered passive/sedentary or (ii) lower than the first reference value, the subject is considered elite/professional or (iii) lower than the second reference value and higher than the first reference value, the subject is considered active/acceptable.

In a particular embodiment, the reference value is obtained from one or more subjects with the same gender as the subject.

According to the first method of the invention, "higher" means at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

According to the first method of the invention, "lower" means at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

*Second method for determining the fitness of a subject*

In a second aspect, the invention relates to an *in vitro* method for determining the fitness of a subject that comprises:

(iii) determining the expression levels of one or more genes selected from the group consisting of GZMK, SNORA38B, and IGF2R, in a sample from said subject, said sample selected from blood, blood plasma and serum, and
(iv) comparing the expression levels obtained in (i) with a reference value, wherein

if the reference value is obtained from one or more elite/professional subjects,

- an increased or equal expression level of at least one of GZMK and SNORA38B genes compared to the reference value, and/or a decreased or equal expression level of IGF2R, gene compared to the reference value indicates that the fitness of the subject is elite/professional, and
- a decreased expression level of at least one of GZMK and SNORA38B genes compared to the reference value, and/or an increased expression level of IGF2R gene compared to the reference value indicates that the fitness of the subject is active/acceptable or passive/sedentary

if the reference value is obtained from one or more active or acceptable subjects,

- a decreased or equal expression level of at least one of GZMK and SNORA38B genes compared to the reference value, and/or an increased or equal expression level of IGF2R gene compared to the reference value indicates that the fitness of the subject is active/acceptable or passive/sedentary, and
- an increased expression level of at least one of GZMK and SNORA38B genes compared to the reference value, and/or a decreased expression level of IGF2R gene compared to the reference value indicates that the fitness of the subject is elite/professional and

if the reference value is obtained from one or more passive/sedentary subjects,

- an increased expression level of at least one of GZMK and SNORA38B genes compared to the reference value, and/or a decreased expression level of IGF2R gene compared to the reference value indicates that the fitness of the subject is elite/professional or active/acceptable, and
- a decreased or equal expression level of at least one of GZMK and SNORA38B genes compared to the reference value, and/or an increased or equal expression level of IGF2R gene compared to the reference value indicates that the fitness of the subject is passive/sedentary.

In a particular embodiment of the second aspect, the *in vitro* method for determining the fitness of a subject that comprises

(i) determining the expression levels of one or more genes selected from the group consisting of GZMK, SNORA38B, and IGF2R, and additionally one or more genes of LILRA6, KLF7, SPATA2, RAB19, and ATP6V0D1 in a sample from said subject, and

(ii) comparing the expression levels obtained in (i) with a reference value, wherein

if the reference value is obtained from one or more elite/professional subjects,

- an increased or equal expression level of at least one of GZMK and SNORA38B genes compared to the reference value, and/or a decreased or equal expression level of at least one of IGF2R, LILRA6, KLF7, SPATA2, RAB19 and ATP6V0D1 genes compared to the reference value indicates that the fitness of the subject is elite/professional, and
- a decreased expression level of at least one of GZMK and SNORA38B genes compared to the reference value, and/or an increased expression level of at least one of IGF2R, LILRA6, KLF7, SPATA2, RAB19 and ATP6V0D1 genes compared to the reference value indicates that the fitness of the subject is active/acceptable or passive/sedentary

if the reference value is obtained from one or more active or acceptable subjects,

- a decreased or equal expression level of at least one of GZMK and SNORA38B genes compared to the reference value, and/or an increased or equal expression level of at least one of IGF2R, LILRA6, KLF7, SPATA2, RAB19 and ATP6V0D1 genes compared to the reference value indicates that the fitness of the subject is active/acceptable or passive/sedentary, and
- an increased expression level of at least one of GZMK and SNORA38B genes compared to the reference value, and/or a decreased expression level of at least one of IGF2R, LILRA6, KLF7, SPATA2, RAB19 and ATP6V0D1 genes compared to the reference value indicates that the fitness of the subject is elite/professional and

if the reference value is obtained from one or more passive/sedentary subjects,

- an increased expression level of at least one of GZMK and SNORA38B genes compared to the reference value, and/or a decreased expression level of at least one of IGF2R, LILRA6, KLF7, SPATA2, RAB19 and ATP6V0D1 genes compared to the reference value indicates that the fitness of the subject is elite/professional or active/acceptable, and
- a decreased or equal expression level of at least one of GZMK and SNORA38B genes compared to the reference value, and/or an increased or equal expression level of at least one of IGF2R, LILRA6, KLF7, SPATA2, RAB19 and ATP6V0D1 genes compared to the reference value indicates that the fitness of the subject is passive/sedentary.

This description discloses also an *in vitro* method for determining the fitness of a subject that comprises (i) determining the expression levels of one or more genes selected from the group consisting of GZMK, SNORA38B, IGF2R, LILRA6, KLF7, SPATA2, RAB19, and ATP6V0D1 in a sample from said subject, and (ii) comparing the expression levels obtained in (i) with a reference value, as indicated above.

The terms *"in vitro"*, "fitness", "subject", "expression levels", "GZMK", "SNORA38B", "IGF2R", "LILRA6", "KLF7", "SPATA2", "RAB19", "ATP6V0D1", "sample", "reference value", "elite or professional" and "active or acceptable" have been defined in connection with the first method of the invention. All the particular and preferred embodiments of the first method of the invention related with these terms apply to the second method of the invention.

In a preferred embodiment, the second method of the invention comprises determining the expression level of SNORA38B.

In a particular embodiment, the second method of the invention further comprises determining the expression levels of one or more genes selected from the group consisting of SPRY3, GPR97, SNORD116-24, SLC43A2, ZSWIM6 and LOC399715. In this particular embodiment:

if the reference value is obtained from one or more elite/professional subjects,

- a decreased or equal expression level of at least one of SPRY3, GPR97, SLC43A2, ZSWIM6 and LOC399715 genes compared to the reference value, and/or an increased or equal expression of SNORD116-24 gene compared to the reference value indicates that the fitness of the subject is elite/professional and
- an increased expression level of at least one of SPRY3, GPR97, SLC43A2, ZSWIM6 and LOC399715 genes compared to the reference value, and/or a decreased expression of SNORD116-24 gene compared to the reference value indicates that the fitness of the subject is active/acceptable or passive/sedentary;

if the reference value is obtained from one or more active/acceptable subjects,

- an increased or equal expression level of at least one of SPRY3, GPR97, SLC43A2, ZSWIM6 and LOC399715 genes compared to the reference value, and/or a decreased or equal expression of SNORD116-24 gene compared to the reference value indicates that the fitness of the subject is active/acceptable or passive/sedentary, and
- a decreased expression level of at least one of SPRY3, GPR97, SLC43A2, ZSWIM6 and LOC399715 genes compared to the reference value, and/or an increased expression of SNORD116-24 gene compared to the reference value indicates that the fitness of the subject is elite/professional;

if the reference value is obtained from one or more passive/sedentary subjects,

- a decreased expression level of at least one of SPRY3, GPR97, SLC43A2, ZSWIM6 and LOC399715 genes compared to the reference value, and/or an increased expression of SNORD116-24 gene compared to the reference value indicates that the fitness of the subject is active/acceptable or elite/professional, and
- an increased or equal expression level of at least one of SPRY3, GPR97, SLC43A2, ZSWIM6 and LOC399715 genes compared to the reference value, and/or a decreased or equal expression of SNORD116-24 gene compared to the reference value indicates that the fitness of the subject is passive/sedentary.

In a particular embodiment, the subject is an athlete. In a more particular embodiment, the athlete is a runner. In an even more particular embodiment, the athlete is an endurance runner. In a still even more particular embodiment, the endurance runner is an ultra-trail runner.

The second method of the invention comprises comparing the expression level of one or more of the genes GZMK, SNORA38B, IGF2R, LILRA6, KLF7, SPATA2, RAB19 and ATP6V0D1 with a reference value.

In a particular embodiment, the reference value is obtained from one or more subjects with the same gender as the subject.

According to the second method of the invention, the expression level of a gene is considered "decreased" when the expression level of said gene in a sample is lower than its reference value. The expression level of a gene is considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

Likewise, in the context of the second method of the invention, the expression level of a gene is considered "increased" when the expression of said marker in a sample is higher than its reference value. The expression level of a gene is considered to be higher than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

According to the second method of the invention, the expression level of a gene is considered "equal" or "substantially equal" if it is less than 5%, less than 4%, less than 3%, less than 2%, less than 1%, less than 0,5%, less than 0,1%, less than 0,05%, less than 0,01%, less than 0,001%, higher or lower than its reference value.

In a particular embodiment of the second method of the invention, the expression levels of GZMK, SNORA38B, IGF2R, LILRA6, KLF7, SPATA2, RAB19 and ATP6V0D1 genes are determined, and the increased expression level of GZMK and SNORA38B genes compared to the reference value and the decreased expression level of IGF2R, LILRA6, KLF7, SPATA2, RAB19 and ATP6V0D1 genes compared to the reference value indicates that the fitness of the subject is elite or professional, and the decreased or equal expression level of GZMK and SNORA38B genes compared to the reference value and the increased or equal expression level of IGF2R, LILRA6, KLF7, SPATA2, RAB19 and ATP6V0D1 genes compared to the reference value indicates that the fitness of the subject is active or acceptable.

In a particular embodiment, the expression level of the at least one gene is determined by measuring the levels of the transcriptional product of said gene (messenger RNA when the gene is a protein-coding gene). In a more particular embodiment, the expression level of the at least one gene is determined by RT-PCR. In another particular embodiment, the expression level of the at least one gene is determined by an oligonucleotide microarray.

*Method for selecting a subject as having a fitness level suitable to participate in an endurance sport*

In a third aspect the invention relates to an *in vitro* method for selecting a subject as having a fitness level suitable to participate in an endurance sport that comprises determining the fitness of the subject using the method of the first aspect or the method of the second aspect, wherein if the fitness level is active/acceptable or elite/professional the subject has a fitness level suitable to participate in an endurance sport.

The terms *"in vitro"*, "subject", "fitness level", "expression level", "sample", "GZMK", "SNORA38B", "IGF2R", "LILRA6", "KLF7", "SPATA2", "RAB19" and "ATP6V0D1", "reference value", "active/acceptable" and "elite/professional" have been defined in connection with the first method of the invention. All the particular and preferred embodiments of the first method of the invention related with these terms apply to the third method of the invention.

The terms "increased expression", "decreased expression" and "equal expression" have been defined in connection with the second method of the invention. All the particular and preferred embodiments of the second method of the invention related with these terms apply to the third method of the invention.

In a preferred embodiment, the third method of the invention comprises determining the expression level of GZMK.

In a particular embodiment, the third method of the invention further comprises determining the expression levels of one or more genes selected from the group consisting of SPRY3, GPR97, SNORD116-24, SLC43A2, ZSWIM6 and LOC399715.

The term "endurance sport" or "endurance exercise", as used herein, refers to a sport in which the athlete exercises the key muscles at submaximal intensity for prolonged periods of time. A sport is considered to be an endurance sport if the athlete practicing it has a metabolic equivalent of task (MET) of more than 6 METs during the practicing. Illustrative non-limitative examples of endurance sports are endurance or long-distance running, swimming, cycling, skiing, race walking, etc. The term "metabolic equivalent of task" or "metabolic equivalent" or "MET", as used herein, refers to a physiological measure of the energy cost of a physical activity and is defined as the ratio of metabolic rate during a specific physical activity to a reference metabolic rate, set by convention to 3.5 ml $O_2 \cdot kg^{-1} \cdot min^{-1}$. Still another definition of 1 MET is 58.2 $W/m^2$, which is equal to the rate of energy produced per unit surface area of an average person seated at rest. The surface area of an average person is 1.8 $m^2$. Metabolic rate is usually expressed in terms of unit area of the total body surface.

In a particular embodiment, the endurance sport is endurance running. In an even more particular embodiment, the endurance running is ultra-trail runner. The terms "endurance running" and "ultra-trail running" have been defined in connection with the first method of the invention. All the particular and preferred embodiments of the first method of the invention related with these terms apply to the third method of the invention.

In a particular embodiment, the subject is an athlete. In a more particular embodiment, the athlete is a runner. In an even more particular embodiment, the athlete is an endurance runner. In a still even more particular embodiment, the endurance runner is an ultra-trail runner.

In a particular embodiment, the third method of the invention comprises determining the fitness level of the subject using the method of the first aspect. In this embodiment, the method comprises:

(i) determining the the expression level of at least one gene selected from the group consisting of GZMK, SNORA38B, and IGF2R, and additionally determining the the expression of one or more gene selected from the group consisting of LILRA6, KLF7, SPATA2, RAB19, and ATP6V0D1 in a sample from said subject, and
(ii) calculating a predictive factor based on the expression levels of said genes, wherein said expression levels are corrected by a particular coefficient for each of the genes,

wherein said predictive value is indicative of the fitness of the subject, wherein if the fitness level of the subject is active/acceptable or elite/professional, the subject has a fitness level suitable to participate in an endurance sport.

In a particular embodiment, the third method of the invention comprises determining the fitness of the subject using the method of the first aspect. In a more particular embodiment, the reference value is obtained from one or more elite or professional subjects and one or more active or acceptable subjects, and in case of coding an elite/professional subject with a numeric value lower than that used for the active/acceptable subject, if the predictive factor is lower than the reference value, the subject is considered elite/professional, or in case of coding an active/acceptable with a numeric value higher than that used for the passive/sedentary, if the predictive factor is higher than the reference value, the subject is considered active/acceptable, and so the subject has a fitness level suitable to participate in an endurance sport. In another more particular embodiment, the reference value is obtained from one or more elite or professional subjects and one or more passive or sedentary subjects and, in case of coding an elite/professional with a numeric value lower than that used for the passive/sedentary, if the predictive factor is lower than the reference value, the subject is considered elite/professional, or in case of coding an elite/professional with a numeric value higher than that used for the passive/sedentary, if the predictive factor is higher than the reference value, the subject is considered elite/professional, so the subject has a fitness level suitable to participate in an endurance sport. In another more particular embodiment, the reference value is obtained from one or more active or acceptable subjects and one or more passive or sedentary subjects. In a particular embodiment, in case of coding an active/acceptable with a numeric value lower than that used for the passive/sedentary, if the predictive factor is lower than the reference value, the subject is considered active/acceptable, or in case of coding an active/acceptable with a numeric value higher than that used for the passive/sedentary, if the predictive factor is higher than the reference value, the subject is considered active/acceptable, so the subject has a fitness level suitable to participate in an endurance sport.

In another particular embodiment, the third method of the invention comprises determining the fitness level of the subject using the method of the second aspect. In this embodiment if the reference value is obtained from one or more elite/professional subjects, an increased or equal expression level of at least one of GZMK and SNORA38B genes compared to the reference value, and/or a decreased or equal expression level of at least one of IGF2R, LILRA6, KLF7, SPATA2, RAB19 and ATP6V0D1 genes compared to the reference value indicates that the fitness of the subject is elite/professional, if the reference value is obtained from one or more active or acceptable subjects an increased expression level of at least one of GZMK and SNORA38B genes compared to the reference value, and/or a decreased expression level of at least one of IGF2R, LILRA6, KLF7, SPATA2, RAB19 and ATP6V0D1 genes compared to the reference value indicates that the fitness of the subject is elite/professional, and if the reference value is obtained from one or more passive/sedentary subjects, an increased expression level of at least one of GZMK and SNORA38B genes compared to the reference value, and/or a decreased expression level of at least one of IGF2R, LILRA6, KLF7, SPATA2, RAB19 and ATP6V0D1 genes compared to the reference value indicates that the fitness of the subject is elite/professional or active/acceptable, so the subject has a fitness level suitable to participate in an endurance sport.

In an even more particular embodiment, the third method comprises determining the fitness level of the subject using the second method of the invention which further comprises determining the expression levels of one or more genes selected from the group consisting of SPRY3, GPR97, SNORD116-24, SLC43A2, ZSWIM6 and LOC399715. In this particular embodiment, the reference value is preferably obtained from one or more elite/professional subjects or one or more active/acceptable subjects. In this particular embodiment, if the reference value is obtained from one or more elite/professional subjects, a decreased or equal expression level of at least one of SPRY3, GPR97, SLC43A2, ZSWIM6 and LOC399715 genes compared to the reference value, and/or an increased or equal expression of SNORD116-24 gene compared to the reference value indicates that the fitness of the subject is elite/professional and if the reference value is obtained from one or more active/acceptable subjects, a decreased expression level of at least one of SPRY3, GPR97, SLC43A2, ZSWIM6 and LOC399715 genes compared to the reference value, and/or an increased expression of SNORD116-24 gene compared to the reference value indicates that the fitness of the subject is elite/professional, so the subject has a fitness level suitable to participate in an endurance sport.

In a particular embodiment, the expression level of the at least one gene is determined by measuring the levels of the transcriptional product of said gene (messenger RNA when the gene is a protein-coding gene). In a more particular embodiment, the expression level of the at least one gene is determined by RT-PCR.

Forms also part of this description a method of determining the fitness level of the subject using the method of the first aspect. In this embodiment, the method comprises:

(iii) determining the the expression level of at least one gene selected from the group consisting of GZMK, SNORA38B, IGF2R, LILRA6, KLF7, SPATA2, RAB19, and ATP6V0D1 in a sample from said subject, and
(iv) calculating a predictive factor based on the expression levels of said genes, wherein said expression levels are corrected by a particular coefficient for each of the genes,

wherein said predictive value is indicative of the fitness of the subject, wherein if the fitness level of the subject is active/acceptable or elite/professional, the subject has a fitness level suitable to participate in an endurance sport.

*Method for monitoring the response of a subject to a training program aimed at improving the fitness of a subject*

In a fourth aspect, the invention relates to an *in vitro* method for monitoring the response of a subject to a training program aimed at improving fitness of a subject that comprises

(i) determining the expression levels of one or more genes selected from the group consisting of GZMK, SNORA38B, and IGF2R, in a sample from said subject, said sample selected from blood, blood plasma and serum, during or after the training program, and
(ii) comparing the expression levels obtained in (i) with the levels of said gene(s) in a sample from the same subject, said sample selected from blood, blood plasma and serum, in a previous point of time,

wherein an increased expression level of at least one of GZMK and SNORA38B genes, and/or a decreased expression level of IGF2R gene compared to the levels in the sample from a previous point of time indicates that the subject has a good response to training, and
wherein a decreased expression level or a lack of change of at least one of GZMK and SNORA38B genes, and/or an increased expression level or a lack of change of IGF2R gene compared to the levels in the sample from a previous point of time indicates that the subject has a bad response to training.

In a particular embodiment of the fourth aspect, the *in vitro* method for monitoring the response of a subject to a training program aimed at improving fitness of a subject that comprises

(i) determining the expression levels of one or more genes selected from the group consisting of GZMK, SNORA38B, and IGF2R, and additionally one or more genes selected from the group consisting of LILRA6, KLF7, SPATA2, RAB19 and ATP6V0D1 in a sample from said subject during or after the training program, and

(ii) comparing the expression levels obtained in (i) with the levels of said gene(s) in a sample from the same subject in a previous point of time,

wherein an increased expression level of at least one of GZMK and SNORA38B genes, and/or a decreased expression level of at least one of IGF2R, LILRA6, KLF7, SPATA2, RAB19 and ATP6V0D1 genes compared to the levels in the sample from a previous point of time indicates that the subject has a good response to training, and

wherein a decreased expression level or a lack of change of at least one of GZMK and SNORA38B genes, and/or an increased expression level or a lack of change of at least one of IGF2R, LILRA6, KLF7, SPATA2, RAB19 and ATP6V0D1 genes compared to the levels in the sample from a previous point of time indicates that the subject has a bad response to training.

This description discloses *an in vitro* method for monitoring the response of a subject to a training program aimed at improving fitness of a subject that comprises

(i) determining the expression levels of one or more genes selected from the group consisting of GZMK, SNORA38B, IGF2R, LILRA6, KLF7, SPATA2, RAB19 and ATP6V0D1 in a sample from said subject during or after the training program, and

(ii) comparing the expression levels obtained in (i) with the levels of said gene(s) in a sample from the same subject in a previous point of time,

wherein an increased expression level of at least one of GZMK and SNORA38B genes, and/or a decreased expression level of at least one of IGF2R, LILRA6, KLF7, SPATA2, RAB19 and ATP6V0D1 genes compared to the levels in the sample from a previous point of time indicates that the subject has a good response to training, and

wherein a decreased expression level or a lack of change of at least one of GZMK and SNORA38B genes, and/or an increased expression level or a lack of change of at least one of IGF2R, LILRA6, KLF7, SPATA2, RAB19 and ATP6V0D1 genes compared to the levels in the sample from a previous point of time indicates that the subject has a bad response to training.

The terms "subject", "expression levels", "GZMK", "SNORA38B", "IGF2R", "LILRA6", "KLF7", "SPATA2", "RAB19", "ATP6V0D1" and "sample" have been defined in connection with the first method of the invention. All the particular and preferred embodiments of the first method of the invention related with these terms apply to the fourth method of the invention.

The terms "increased expression" and "decreased expression" have been defined in connection with the first method of the invention. All the particular and preferred embodiments of the first method of the invention related with these terms apply to the fourth method of the invention.

The term "training program aimed at improving fitness of a subject" as used herein, refers to a regimen of exercises designed to increase or improve the fitness of a subject. The training can consist on one or more sessions of variable duration, for example, one, two, three, four, five, six, seven, eight, nine, ten or more sessions of. The training can also consist on a number of sessions over a period of time, for example, daily sessions, or once, twice, three times, four times, five times, six times, seven times a week, etc. in a particular embodiment, the training program is an endurance training program, that is, aimed at improving the endurance of a subject.

The term "response to training", as used herein, refers to the changes in the fitness of a subject as a result of a training program or regimen. The response of training can be good or bad. A "good response" to training means that the fitness of the subject is improved in response to training and a "bad response" to training means that the fitness of the subject is not significantly improved in response to training. In a particular embodiment, a subject has a good response to training if his fitness level changes from passive/sedentary to active/acceptable or elite/professional in response to training. In another particular embodiment, the subject has a good response to training if his fitness level changes from active/acceptable to elite/professional in response to training. In another particular embodiment, the subject has a bad response to training if his fitness level does not change from passive/sedentary to active/acceptable or elite/professional in response to training. In another particular embodiment, the subject has a bad response to training if his fitness level does not change from active/acceptable to elite/professional in response to training.

The fourth method of the invention comprises determining the expression levels of one or more genes selected from the group consisting of GZMK, SNORA38B, IGF2R, LILRA6, KLF7, SPATA2, RAB19 and ATP6V0D1 in a sample from said subject during or after the training program. The term "during" means that the sample is obtained in the course of the training program. If the training program comprises several sessions, the sample can be obtained during one of the sessions or before or after one of the sessions. The term "after" means that the sample is obtained after the training program is completed, for example, immediately after completing the program or in the hours or days following the end of the training program.

The fourth method of the invention comprises comparing the expression levels obtained in (i) with the levels of said gene(s) in a sample from the same subject in a previous period of time. The expression "previous point of time", as used herein, means that the sample has been obtained in a point of time before the time when the sample in step (i) has been obtained. In a particular embodiment, "previous period of time" means before beginning the training program, or during the training program but before the time the sample in step (i) was obtained.

In a particular embodiment, the method comprises determining the expression levels of GZMK, SNORA38B, IGF2R, LILRA6, KLF7, SPATA2, RAB19 and ATP6V0D1, wherein

- the increased expression level of GZMK and SNORA38B genes compared to levels in the sample from a previous point of time and the decreased expression level of IGF2R, LILRA6, KLF7, SPATA2, RAB19 and ATP6V0D1 genes compared to levels in the sample from a previous point of time indicates that the subject has a good response to training, and
- the decreased or lack of change of the expression level of GZMK and SNORA38B genes compared to the levels in the sample from a previous point of time and the increased or lack of change of the expression level of IGF2R, LILRA6, KLF7, SPATA2, RAB19 and ATP6V0D1 genes compared to levels in the sample from a previous point of time indicates that the subject has a bad response to training.

In a particular embodiment, the method further comprises determining the expression levels of one or more genes selected from the group consisting of SPRY3, GPR97, SNORD116-24, SLC43A2, ZSWIM6, and LOC399715 in a sample from said subject. In this particular embodiment,

- the decreased expression level of SPRY3, GPR97, SLC43A2, ZSWIM6 and LOC399715 genes compared to levels in the sample from a previous point of time and/or the increased expression level of SNORD116-24 gene compared to levels in the sample from a previous point of time indicates that the subject has a good response to training and
- the increased or lack of change of the expression level of SPRY3, GPR97, SLC43A2, ZSWIM6 and LOC399715 genes compared to t levels in the sample from a previous point of time and/or the decreased or lack of change of the expression level of SNORD116-24 gene compared to levels in the sample from a previous point of time indicated that the subject has a bad response to training.

In a particular embodiment, the sample is blood.

In a particular embodiment, the gene expression levels are determined by RT-PCR or an oligonucleotide microarray.

*Method for selecting a personalized training program aimed at improving fitness of a subject*

In a fifth aspect, the invention relates to an *in vitro* method for selecting a personalized training program aimed at improving fitness of a subject that comprises

(i) determining the expression levels of one or more genes selected from the group consisting of GZMK, SNORA38B, and IGF2R, in a sample from said subject, said sample selected from blood, blood plasma and serum, during or after the training program under test, and
(ii) comparing the expression levels obtained in (i) with the levels of a sample from the same subject, said sample selected from blood, blood plasma and serum, before starting the training program under test,

wherein the training program under test is selected if there is an increased expression level of at least one of GZMK and SNORA38B genes, and/or a decreased expression level of IGF2R gene compared to the level of expression before starting the training program, and
wherein the training program under test is not selected if there is a decreased expression level or a lack of change of at least one of GZMK and SNORA38B genes, and/or an increased expression level or a lack of change of IGF2R gene compared to the level of expression before starting the training program.

In a particular embodiment of the fifth aspect, the *in vitro* method for selecting a personalized training program aimed at improving fitness of a subject that comprises

(iii) determining the expression levels of one or more genes selected from the group consisting of GZMK, SNORA38B, and IGF2R, and additionally one or more of the genes selected from the group consisting of LILRA6, KLF7, SPATA2, RAB19 and ATP6V0D1 in a sample from said subject during or after the training program under test, and

(iv) comparing the expression levels obtained in (i) with the levels of a sample from the same subject before starting the training program under test,

wherein the training program under test is selected if there is an increased expression level of at least one of GZMK and SNORA38B genes, and/or a decreased expression level of at least one of IGF2R, LILRA6, KLF7, SPATA2, RAB19 and ATP6V0D1 genes compared to the level of expression before starting the training program, and

wherein the training program under test is not selected if there is a decreased expression level or a lack of change of at least one of GZMK and SNORA38B genes, and/or an increased expression level or a lack of change of at least one of IGF2R, LILRA6, KLF7, SPATA2, RAB19 and ATP6V0D1 genes compared to the level of expression before starting the training program.

This description shows an *in vitro* method for selecting a personalized training program aimed at improving fitness of a subject that comprises

(v) determining the expression levels of one or more genes selected from the group consisting of GZMK, SNORA38B, IGF2R, LILRA6, KLF7, SPATA2, RAB19 and ATP6V0D1 in a sample from said subject during or after the training program under test, and

(vi) comparing the expression levels obtained in (i) with the levels of a sample from the same subject before starting the training program under test,

wherein the training program under test is selected if there is an increased expression level of at least one of GZMK and SNORA38B genes, and/or a decreased expression level of at least one of IGF2R, LILRA6, KLF7, SPATA2, RAB19 and ATP6V0D1 genes compared to the level of expression before starting the training program, and

wherein the training program under test is not selected if there is a decreased expression level or a lack of change of at least one of GZMK and SNORA38B genes, and/or an increased expression level or a lack of change of at least one of IGF2R, LILRA6, KLF7, SPATA2, RAB19 and ATP6V0D1 genes compared to the level of expression before starting the training program.

The terms "subject", "expression levels", "GZMK", "SNORA38B", "IGF2R", "LILRA6", "KLF7", "SPATA2", "RAB19", "ATP6V0D1" and "sample" have been defined in connection with the first method of the invention. All the particular and preferred embodiments of the first method of the invention related with these terms apply to the fifth method of the invention.

The terms "increased expression" and "decreased expression" have been defined in connection with the first method of the invention. All the particular and preferred embodiments of the first method of the invention related with these terms apply to the fifth method of the invention.

The term "training program aimed at improving fitness of a subject" has been defined been defined in connection with the fourth method of the invention. All the particular and preferred embodiments of the fourth method of the invention related with these terms apply to the fifth method of the invention.

The fifth method of the invention comprises determining the expression levels of one or more genes selected from the group consisting of GZMK, SNORA38B, IGF2R, LILRA6, KLF7, SPATA2, RAB19 and ATP6V0D1 in a sample from said subject during or after the training program under test. The terms "during" and "after" have been previously defined.

The fifth method of the invention comprises comparing the expression levels obtained in (i) with the levels of said gene(s) in a sample from the same obtained before starting the training program. The term "before", as used herein, means that the sample has been obtained in a point of time before staring the training program, for example, immediately before staring the program or in the hours or days previous to the beginning of the training program.

In a particular embodiment, the method comprises determining the expression levels of GZMK, SNORA38B, IGF2R, LILRA6, KLF7, SPATA2, RAB19 and ATP6V0D1, wherein

- the training program under test is selected if there is an increased expression level of GZMK and SNORA38B genes, and a decreased expression level of IGF2R, LILRA6, KLF7, SPATA2, RAB19 and ATP6V0D1 genes compared to the level of expression before starting the training program, and

- the training program under test is not selected if there is a decreased expression level or a lack of change of GZMK and SNORA38B genes, and an increased expression level or a lack of change of IGF2R, LILRA6, KLF7, SPATA2, RAB19 and ATP6V0D1 genes compared to the level of expression before starting the training program.

In a particular embodiment, the method further comprises determining the expression levels of one or more genes selected from the group consisting of SPRY3, GPR97, SNORD116-24, SLC43A2, ZSWIM6, and LOC399715 in a sample from said subject. In this particular embodiment,

- the training program under test is selected if there is a decreased expression level of SPRY3, GPR97, SLC43A2, ZSWIM6 and LOC399715 genes compared to the level of expression before starting the training program and/or increased expression level of SNORD116-24 gene compared to the level of expression before starting the training program and
- the training program under test is not selected if there is an increased expression level of SPRY3, GPR97, SLC43A2, ZSWIM6 and LOC399715 genes compared to the level of expression before starting the training program and/or a decreased expression level of SNORD116-24 gene compared to the level of expression before starting the training program.

In a particular embodiment, the sample is blood.

In a particular embodiment, the gene expression levels are determined by RT-PCR or an oligonucleotide microarray.

*Kit of the invention and uses thereof*

Although not forming part of the invention, disclosed herewith is a kit comprising a set of reagents capable of determining the expression levels of at least two genes selected from the group consisting of GZMK, SNORA38B, and IGF2R, wherein said reagents are oligonucleotides that specifically hybridize with the transcriptional product of the genes or compounds that specifically bind to the protein encoded by the genes; and wherein said reagents comprise at least the 10 % of the total amount of reagents for assaying gene expression markers forming the kit.

This description refers also to a kit comprising a set of reagents capable of determining the expression levels of at least two genes selected from the group consisting of GZMK, SNORA38B, IGF2R, LILRA6, KLF7, SPATA2, RAB19 and ATP6V0D1 genes.

The term "kit" or "kit-of-parts", as used herein, relates to an entity of physically separated components, which are intended for individual use, but in functional relation to each other. The kit is a product that contains the different reagents needed to carry on the different methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kit can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

In a particular embodiment, the kit further comprises reagents for determining the expression levels of at least one gene selected from the group consisting of SPRY3, GPR97, SNORD116-24, SLC43A2, ZSWIM6, and LOC399715.

The terms "expression levels", "GZMK", "SNORA38B", "IGF2R", "LILRA6", "KLF7", "SPATA2", "RAB19", "ATP6V0D1", "SPRY3", "GPR97", "SNORD116-24", "SLC43A2", "ZSWIM6", and "LOC399715" have been defined in connection to the first method of the invention.

In a preferred embodiment, the kit comprises a set of reagents capable of determining the expression level of GZMK.

In another particular embodiment, the kit comprises a set of reagents capable of determining the expression levels of GZMK, SNORA38B, and IGF2R, said reagents being the only reagents for assaying gene expression markers forming the kit.

In another particular embodiment, the kit comprises a set of reagents capable of determining the expression levels of GZMK, SNORA38B, IGF2R, LILRA6, KLF7, SPATA2, RAB19 and ATP6V0D1, wherein said reagents are oligonucleotides that specifically hybridize with the transcriptional product of the genes or compounds that specifically bind to the protein encoded by the genes; and wherein said reagents comprise at least the 10 % of the total amount of reagents for assaying gene expression markers forming the kit. In a more particular embodiment, the kit comprises a set of reagents capable of determining the expression levels of GZMK, SNORA38B, IGF2R, LILRA6, KLF7, SPATA2, RAB19 and ATP6V0D1; and the said reagents are the only reagents for assaying gene expression markers forming the kit.

The term "reagents capable of determining the expression levels", as used herein, refers to reagents capable of determining the transcriptional product of the gene (mRNA in case of protein-coding genes) or the protein encoded by said gene.

In a particular embodiment, said reagents are oligonucleotides that specifically hybridize with the transcriptional product of the gene (mRNA in case of protein coding genes). The oligonucleotide can be a primer or a probe.

In an embodiment, said reagents are a set of one or more pairs of oligonucleotide primers designed to specifically amplify the nucleic acids of the invention.

Probes can be used for determining the expression level by means of PCR. A probe is an oligonucleotide having a defined sequence capable of specifically hybridizing with a complementary sequence of a nucleic acid, so it can be used for detecting and identifying complementary or substantially complementary sequences in nucleic acids. The length of the probe of the invention can vary within a large range although for practical reasons, probes having a small length of less than 10 nucleotides are preferred, preferably at least 15 nucleotides, preferably at least 20 nucleotides, preferably at least 25 nucleotides and, preferably not more than 100 nucleotides, more preferably comprised between 15 bases and 30 bases, preferably between 16 bases and 22 bases.

Alternatively or additionally, the oligonucleotides used can contain modified bonds such as phosphodiester, phosphotriester, phosphorothioate, phosphorodithioate, phosphoroselenoate, phosphorodiselenoate, phosphoroanilothioate, phosphoroamidate, methylphosphonate, boranophosphonate bonds, as well as combinations thereof, or they are peptide nucleic acids (PNAs), in which the different nucleotides are bound by amide bonds.

In case that the oligonucleotide is a probe, said probe can have at its 3' end a fluorophore and at its 5' end a molecule blocking its fluorescence emission (quencher). Said probe hybridizes specifically in the central part of the PCR product to be obtained.

Although not forming part of the invention, a kit containing a microarray that has probe oligonucleotides which specifically recognize the transcriptional products of the genes (mRNA in case of protein-coding genes) is also disclosed. The microarrays contained in the kit may also include probe oligonucleotides which specifically recognize RNAs suitable for normalization purposes. In certain embodiments the kit further contains a control sample. This sample can be derived from a control or normal cell, tissue, or subject, e.g., one that exhibits, for example, normal traits. In additional embodiments, the kits contain instructions for use.

The expression "hybridize specifically" refers to such conditions that allow the hybridization of two polynucleotides of single chain, of complementary sequence or having a high grade of similarity, under stringent conditions or moderately stringent conditions, producing a double chain single molecule by base pairing.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995). Stringent conditions and moderately stringent conditions are identified as described by Sambrook et al., Molecular Cloning: A Laboratory Manual, New York: Cold Spring Harbor Press, 1989.

The kits also contain other reagents that allow determining the expression level of a gene but that are not specific for said gene, e.g. reagents for the extraction of RNA material, etc., e.g., primers for the synthesis of the corresponding cDNA by means of RT, reagents for the amplification of DNA such as DNA polymerases, dNTPs, buffers, etc.

In a particular embodiment, said reagents are compounds that specifically bind to the protein encoded by the gene. In a more particular embodiment, said reagents are antibodies, aptamers or fragments thereof.

The antibodies can be fixed to a solid support such as a membrane, a plastic or a glass, optionally treated to facilitate the fixation of said antibodies to the support. Said solid support comprises, at least, a set of antibodies which specifically recognize the marker, and which can be used for detecting the levels of expression of said marker.

Additionally, the kits comprise reagents for detecting the expression levels of a constitutive gene. Said additional reagents allows normalizing the measurements performed in different samples (for example, the sample to be analyzed and the control sample) to rule out that the differences in the expression of the genes are due to a different quantity of total DNA amount in the sample more than the real differences in the relative levels of expression. The constitutive genes in the present invention are genes that are always active or being transcribed constantly and which encode for proteins that are expressed constitutively and carry out essential cellular functions, including without limitation, $\beta$-2-microglobulin (B2M), ubiquitin, 18-S ribosomal protein, cyclophilin, GAPDH, PSMB4, tubulin and actin.

In a preferred embodiment, the reagents capable of determining the expression levels of the genes comprise at least 10%, at least 20%, at least 30%, at least 40%, at least 50%, at least 60%, at least 70%, at least 80%, at least 90% or at least 100% of the total amount of reagents for assaying biomarkers forming the kit. Thus, in the particular case of kits comprising reagents for determining the expression levels of GZMK, SNORA38B, IGF2R, LILRA6, KLF7, SPATA2, RAB19 and ATP6V0D1, the reagents specific for said genes comprise at least 10%, at least 20%, at least 30%, at least 40%, at least 50%, at least 60%, at least 70%, at least 80%, at least 90% or at least 100% of the reagents for detecting biomarkers present in the kit. In the particular case of kits comprising reagents for determining the expression levels of GZMK, SNORA38B, IGF2R, LILRA6, KLF7, SPATA2, RAB19, ATP6V0D1, SPRY3, GPR97, SNORD116-24,

SLC43A2, ZSWIM6, and LOC399715, the reagents specific for said genes comprise at least 10%, at least 20%, at least 30%, at least 40%, at least 50%, at least 60%, at least 70%, at least 80%, at least 90% or at least 100% of the reagents for detecting biomarkers present in the kit.

*Computer program and computer-implemented method*

In a sixth aspect, the invention relates to a computer-implemented method for determining the fitness of a subject according to any one of the first and second aspects, or for selecting a subject as having a fitness level suitable to participate in an endurance sport according to the third aspect, or for monitoring the response to training of a subject during an endurance exercise training according to the fourth aspect, or for selecting a personalized training program aimed at improving fitness of a subject according to the fifth aspect, which computer-implemented method comprises:

- collection of the data of the expression level of the genes determined in methods of aspects first to fifth;
- analyzing the collected data by (a) calculating a predictive factor based on the expression levels of said genes, wherein said expression levels are corrected by a particular coefficient for each of the genes, wherein said predictive factor is indicative of the fitness of the subject, and wherein said coefficient for each of the genes is a coefficient as shown in columns 4 and 5 of Table 3.; or by (b) comparing the expression levels obtained with a reference value; or by (c) comparing the expression levels obtained with the levels of said genes in a previous point of time; or by (d) comparing the expression levels with the levels of said genes, before starting a training program under test; and
- providing a result of the analysis.

In an eight aspect, the invention relates to a computer program for executing the computer-implemented method according to the sixth aspect.

The description includes a computer-implemented method for determining the fitness of a subject according to the first or second aspect, or for selecting a subject as having a fitness level suitable to participate in an endurance sport according to the third aspect, or for monitoring the response to training of a subject during an endurance exercise training according to the fourth aspect, or for selecting a personalized training program aimed at improving fitness of a subject according to the fifth aspect. It is also disclosed a computer program for executing a method according to any aspects first to fifth.

Computer implementation can be achieved using a computer program providing instructions in a computer readable form. The computer would collect the data, analyze the data as in accordance with the methods described herein, and then provide a result of the analysis.

Different types of computer language can be used to provide instructions in a computer readable form. The methods of the invention can be implemented on a stand-alone computer or as part of a networked computer system.

The invention is defined below by virtue of the following examples which are to be construed as merely illustrative and not limitative of the scope of the invention.

**EXAMPLES**

**EXAMPLE 1**

**Materials and Methods**

**Experimental design**

A total of 17 healthy endurance runners were voluntarily recruited to participate in the study (12 male aged $38.2 \pm 4.3$ years, 5 female aged $35.6 \pm 2.2$ years). All individuals were experienced nonprofessional athletes of Western European descent who were specifically trained for UMT. Runners were classified into two different training regime groups based on their weekly exercise hours: (i) Active group (3 to 10 training hours) and (ii) Elite group (more than 10 hours). Participants provided written informed consent.

Experiment was deployed on the "Cavalls del Vent" TUM in June 2012, an official competition organized by Salomon Nature Trails and located in Cadí-Moixeró Natural Park (Catalan Pyrenees, Spain). It was a circular route with a length of approximately 82 km and a total cumulative elevation gain of 12180 m. The start of the race was at 755 m above sea level and the maximum summit achieved during the trail was 2520 m. Adverse weather conditions during the competition caused that only three out of 17 participants completed the race.

**Blood samples and RNA extraction**

Venous blood samples were drawn from the antecubital vein at rest in a sitting position and collected into PAXgene Blood RNA Tubes according to the manufacturer's protocol (PreAnalytiX GmbH/QIAGEN, Switzerland/US). Samples were obtained from each subject in previous periods before the race and immediately after completing their participation in the TUM with the exception of five subjects from whom only pre-race samples were available. A total of 29 samples, 17 of them corresponded to pre-race and 12 to post-race, were stored at -80°C until their assay in Hospital de la Santa Creu I Sant Pau (HSCiSP) (Barcelona, Spain). Samples were tagged with an identifier number followed by PRE or POST referring to pre or post-race sample.

Total RNA was isolated using the PAXgene Blood RNA kit (PreAnalytiX GmbH/QIAGEN, Switzerland/US). The concentration of the extracted RNA was measured spectrophotometrically (Nanodrop 1000/ Thermo Fisher Scientific, Wilmington, US).

**HuGene2.0st expression profiling**

RNA was amplified and biotinylated using the Ambion TotalPrep RNA Amplification kit (Life Technologies, Carlsbad US). The corresponding expression profiles were obtained after RNA was hybridized to HuGene2.0st microarrays (Affymetrix Inc., California US). HSCiSP laboratory carried out the hybridization, washing, staining, scanning and grid alignment processes according to manufacturer's instructions to get raw fluorescence intensity values stored in CEL file types, one per each available blood sample. Expression profiling was acquired between 8th of March 2013 and 19th of April of 2013.

**Microarray data pre-processing and quality control**

All CEL files were pre-processed on the software platform R for Statistical Computing v3.2.0 (Team, R. C., "R: A language and environment for statistical computing. R Foundation for Statistical Computing." Vienna, Austria, 2015) with BioConductor v3.1 (Gentleman, R. C. et al, Genome Biol. 2004, 5 (10): R80). Raw fluorescence intensity values were background corrected, quantile normalized and summarized using the Robust Multichip Average (RMA) (Irizarry, R. A. et al, Biostatistics 2003, 4 (2): 249-264) algorithm implemented in the *oligo* package v1.32.0 (Carvalho, B. S. and Irizarry, R. A., Bioinformatics, 2010, 26(19): 2363-2367). Summarization was performed at transcript cluster level to combine all individual probes interrogating the same gene. The expression levels of 53617 transcript clusters (TCs) were available per sample. Each TC had an eight-digit identifier from the manufacturer.

Quality control (QC) was performed over pre-processed data to detect possible outliers based on the following metrics: relative log expression (RLE) (Bolstad, B. M. et al, Int. Rev. Neurobiol 2004, 60: 25-58), normalized unscaled standard error (NUSE) (Bolstad, B. M. et al, Int. Rev. Neurobiol 2004, 60: 25-58), density intensity distributions (histogram and boxplot) and principal component analysis (PCA). As a result of this QC, one pre-race sample was excluded since it showed a distinct pattern compared to the rest. This outlier has no post-race counterpart. Pre-processing and QC was repeated after its removal with positive results. A total of 28 samples were available for further analysis as it is shown in Table 1.

| Samples distribution for downstream analysis | | | | | |
|---|---|---|---|---|---|
| Before (PRE) or after (POST) race | Gender | | | | TOTAL |
| | Female | | Male | | |
| | Elite | Active | Elite | Active | |
| PRE | 3 | 2 | 3 | 8 | 16 |
| POST | 2 | 1 | 2 | 7 | 12 |

| Total | 8 | 20 | 28 |
|---|---|---|---|
| Age: 38.8 ± 3.3 years | | | |

**Table 1**: Samples distribution for downstream analysis after Quality Control was performed over pre-processed data. Table shows samples distribution among gender, training regime and time point with respect to the race.

**Differential gene expression analysis**

TCs were annotated to respective target genes based on the annotation found in *hugene20sttranscriptcluster.db* package (MacDonald, J. W. "hugene20sttranscriptcluster.db: Affymetrix hugene20 annotation data (chip hugene20sttranscriptcluster). R package version 8.3.1.), so entrez gene IDs and official genes symbols were mapped from TCs identifiers. Only those TCs with any annotation were considered for differential gene expression analysis (DGEA) comprising protein-coding and non-protein-coding genes. A non-supervised filtering (Bourgon, R., Proc. Natl. Acad. Sci. 2010, 107 (21): 9546-9551) was applied to discard TCs with low expression levels and low variability across all samples since they are assumed to be non-informative. It was required to TCs to have expression measurements above 100 fluorescence units in at least 25% of the samples and the interquartile range across the samples (log base 2 scale) to be at least 0.5. The genefilter package v1.50.0 (Gentleman, R. et al, "genefilter: genefilter: methods for filtering genes from high-throughput experiments. R package version 1.50.0.") was used for this purpose.

Through *limma* package v3.24.13 (Ritchie, M. E., Nucleic Acids Res., 2015, 43 (7): e47), a linear regression model (LM) was fit to each TC expression value as shown in Equation (2).

$$g_k \approx \beta_{0k} + \beta_{1k} \cdot pp + \beta_{2k} \cdot t + \beta_{3k} \cdot g + \beta_{4k} \cdot (pp:t) \qquad (2)$$

where $g_k$ is the expression value of TC $k$ , $\beta_{0k}$ is the LM intercept for TC expression value k, $\beta_{1k}$ , $\beta_{2k}$ , $\beta_{3k}$ and $\beta_{4k}$ are the unknown coefficients for variables pre-post race pp, training regime t, gender *g* and an interaction effect between pp and t respectively.

The empirical Bayes moderated t-statistics tested each individual coefficient equal to zero within the limma package. Statistically significant differentially expressed TCs (differential TCs) were selected and ranked (adjusted p-value < 5%, FDR) per each variable on the LM.

**Predictive model construction**

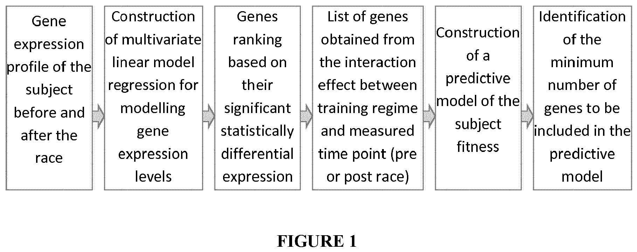

Predictive model was constructed based on the basal gene expression levels from pre-race samples and respective to those DGEA ranked genes from the interaction effect ($\beta_4 \neq 0$, see Eq. 2). Figure 1 shows a general overview of the followed procedure which starts from the gene expression profile data and finishes with the identification of the minimum number of genes to be included in the predictive model.

The *n* differential genes from the interaction effect may represent a number of variables considerably high compared to the number of available observations. In this situation, a LM may adjust reasonably well the available data but may fail to predict new observations due to an overfitting issue. In order to avoid this, a partial least squares regression (PLSR) was implemented for identifying the subset of genes that were most strongly related to the training level or subject fitness as indicated in Equation (1). Package pls v2.4-3 (Bjørn-Helge Mevik et al, "pls: Partial Least Squares and Principal Component regression." 2013) was used for this purpose.

$$\text{Individual\_fitness} \approx w_0 + w_1 \cdot \text{gender} + \sum_{i=2}^{n} w_i \cdot g_i + \epsilon \qquad (2)$$

where Individual_fitness is a qualitative variable which classifies an individual in the elite or active groups, $w_0$ is the PLSR model intercept, $w_1$, and $w_i$ are the unknown weights for variables *gender* and basal gene expression levels $g_i$, respectively; $\epsilon$ is the random error.A t-statistics tested each individual regression coefficient equal to zero within the pls package (H. Martens and M. Martens, Food Qual. Prefer. 2000, 11 (1-2): 5-16).

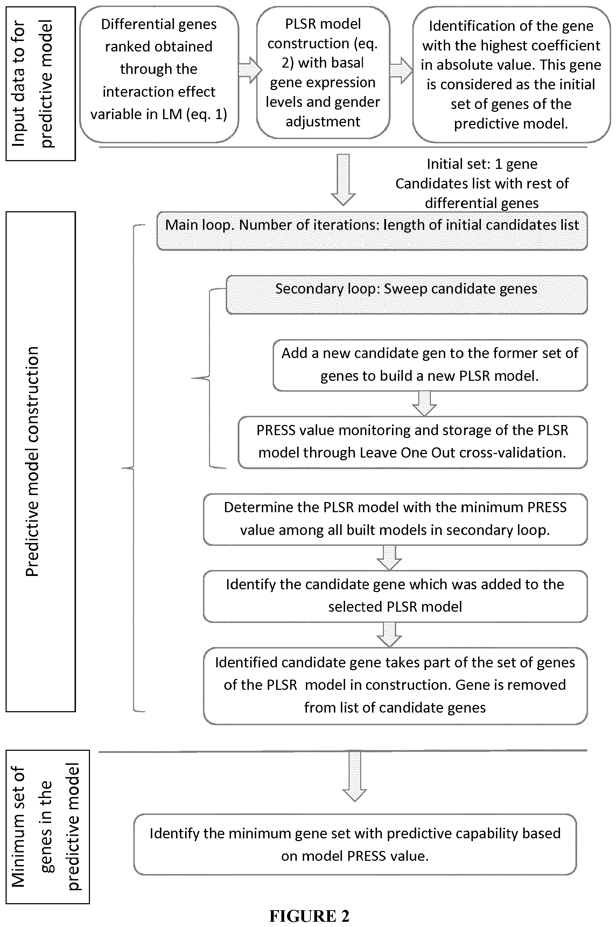

The selection of genes was the result of an iterative process as it is further explained below. Previous to this step, a PLSR model, as formulated in Eq. 1, was adjusted with the complete list of differential genes obtained from the LM interaction effect with no iterative process. The gene with the highest weight was appointed as the first gene to be included in the subset of minimum genes considered in the predictive model. A detailed workflow scheme for the predictive model construction is shown in Figure 2. Leave-one-out (LOO) cross validation was used for model assessment and as a result of it, the Predicted Residual Sum of Squares (PRESS) was used as an indicator of the proper predictions, being the lower the better. The Root Mean Squared Error of Prediction (RMSEP), which is derived from PRESS, is calculated once the iterative process is completed for determining the optimum number of latent variables of the PLSR.

*Iterative process for gene selection*

Following iterative process implemented for identifying the minimum subset of genes with predictive capabilities:

1. Predictive model was initialized with one gene and the variable *gender.* This one gene was selected from a first PLSR model which simultaneously considering all differential genes from the interaction effect in LM. The gene with the highest weight was chosen. A list of candidate genes was built with the unselected ones.
2. For each step of a main iterative process, a gene was added to the predictive model one at a time until all candidate genes were included.

   a. In order to select this specific gene, a secondary iterative process was defined in the following way:

      i. Each of the candidate genes was separately added to the predictive model obtaining a PLSR model with the initial gene or genes (if it is not the first iteration) and a candidate gene.
      ii. For each PLSR model constructed, PRESS values were obtained through LOO cross-validation for all computed latent variables. Minimum PRESS value was determined and saved with the candidate gene label assignation.

   b. The PLSR model with the minimum PRESS value was identified among all built models through the secondary loop. This minimum PRESS value is saved for all computed latent variables for further analysis (step 3).
   c. The candidate gene, associated with the PLSR model selected in previous step, was identified. This became part of the predictive model under construction and was removed from the list of candidates.

3. In order to determine the minimum subset of genes with prediction capability, stores PRESS values were analysed as a function of the number of genes included in the model and for each number of latent variables.

## Results

### 1,792 **TCs are differentially expressed** as a **result of** DGEA

2,850 TCs were available for DGEA after applying the non-supervised filtering to those TCs with any official annotation (23,678 in total among 53,617 included in the microarray). DGEA identified 1,792 statistically significant differentially expressed TCs. *Table 2* shows their distribution per variable included in the LM.

| Number of statistically significant genes showing differential expression per each variable in the LM model as defined in equation (1) | |
| --- | --- |
| Variable | Number of ranked TCs |
| Time point (Pre or Post – race) | 1480 |
| Training regime | 18 |
| Gender | 26 |
| Interaction effect | 268 |

**Table 2**: Distribution of statistically significant differentially expressed TCs, as a result of DGEA, per each variable included in the LM.

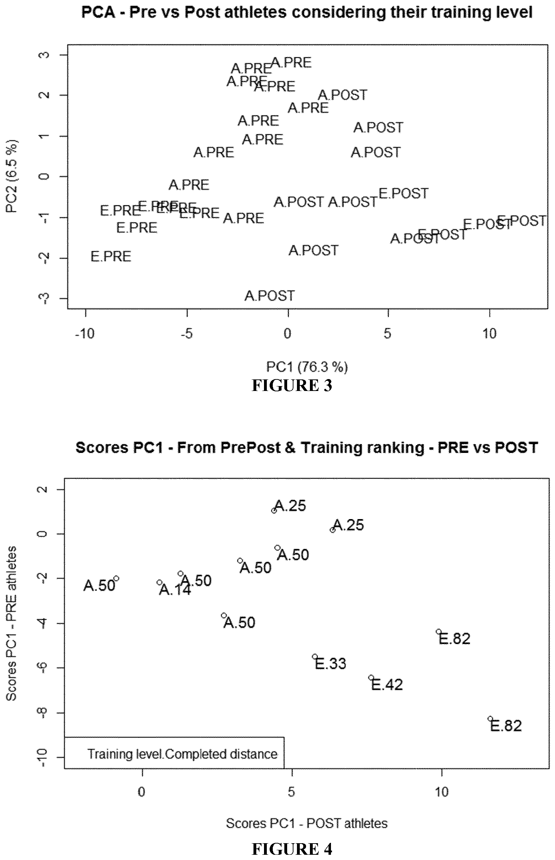

The 268 differential TCs related to the interaction effect are interrogating 259 different genes since there were TCs redundancies for some genes. *Figure 3* shows the first two components of a Principal Component Analysis (PCA) and considering the expression levels of the top 100 (p.val<0.001) among the 268 TCs ranked through the interaction effect as shown in Table 2. Each sample is labelled with the subject training level (E for Elite and A for Active) and the time point of collecting the sample (pre or post-race). It is shown how the first PC was capturing 76.3% of the total data variance. Subjects classified as elite showed a higher dynamic range than the active ones when evaluating their response to exercise.

Figure 4 shows the projections (scores) of each sample over the first PC obtained in Figure 1. Only those subjects with pre and post-race data are considered. Samples are labelled with their training level and the race distance in kilometers they completed. Those elite subjects are all located in the bottom right quadrant while all active subjects are mainly located in the top left, these different locations are independent of the completed distance.

**Predictive model through PLSR**

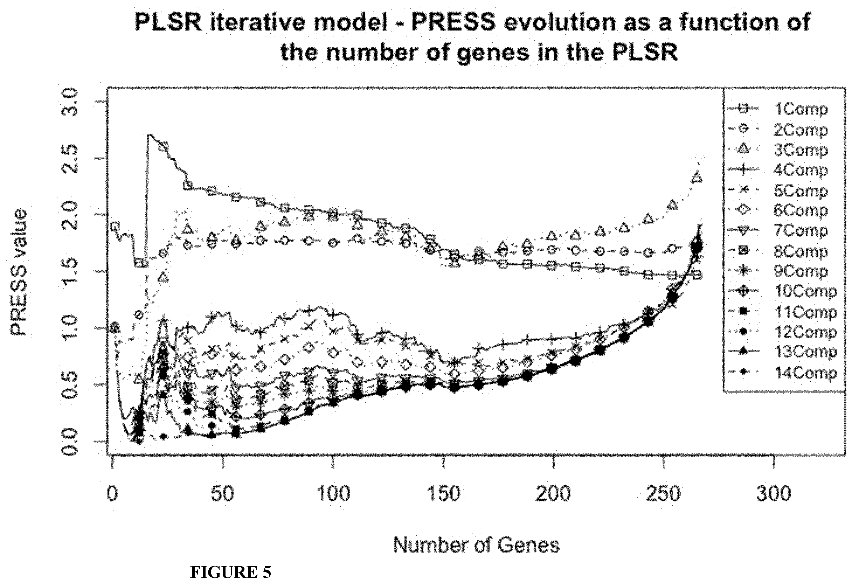

As a result of building a PLSR model with all the 268 ranked TCs obtained from the interaction effect and adjusting by gender, SNORA38B gene is identified as the gene with the highest weight. This gene is considered the first gene to take part of the predictive model and its initialization for the iterative process which was carried out for selecting the rest of genes with prediction capabilities. *Figure 5* shows how the PRESS values evolutions as candidate genes are incorporated to the PLSR model based on the iterative process selection, one at a time.

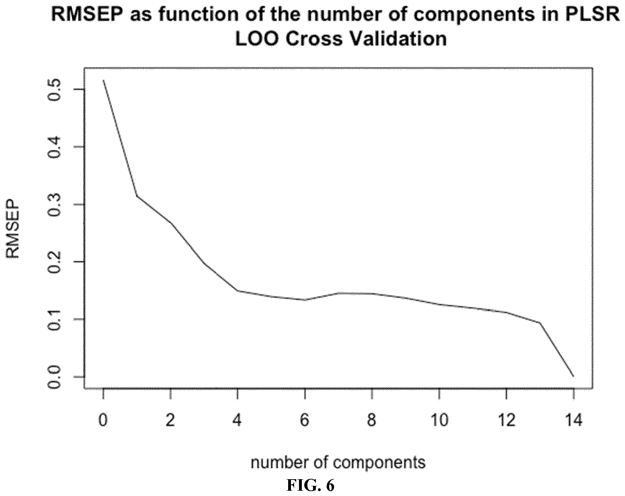

From previous figure, it can be seen that the minimum PRESS values are obtained when a reduced set of genes (below 15 genes). Figure 6 illustrates how the RMSEP changes as the number of latent variables in the PLSR model increases when a reduced of subset of genes is considered. No significant improvement is achieved in RMSEP from five components on.

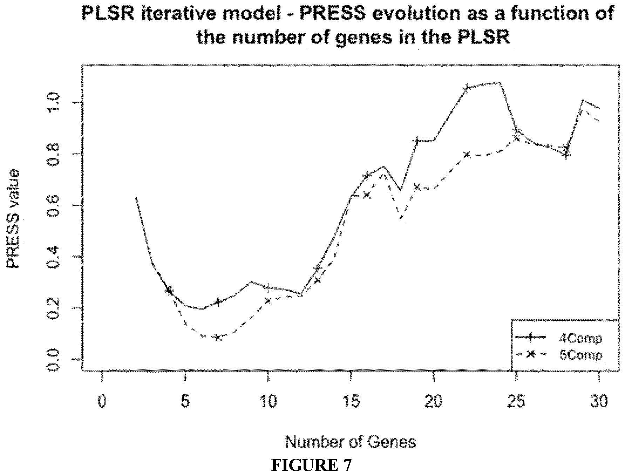

Figure 7 shows a detailed view of the PRESS evolution reported in Figure 5 focusing on four and five latent variables and up to 26 genes in the model. In the case of five latent variables, the lowest PRESS values (<0.25) are obtained up to fourteen genes with an optimum subset of eight genes.

**List of minimum subset of genes with predictive capabilities**

Table 3 shows the list of 14 genes which showed a PRESS value below 0.25 when considered in the predictive model together with gender adjustment.

| Gene Symbol | Gene ID | Transcript Cluster ID | Weight value in optimum predictive model[2] | Weight value in predictive model[3] |
|---|---|---|---|---|
| GZMK[1] | 3003 | 16984783 | 0.6491 *** | 0.5696 *** |
| IGF2R[1] | 3482 | 17014364 | 0.5380 *** | 0.3486 * |
| LILRA6[1] | 79168 | 16875366 | -0.4349 *** | -0.3943 ** |
| SPATA2[1] | 9825 | 16920180 | 0.3999 * | 0.1762 |

| Gene Symbol | Gene ID | Transcript Cluster ID | Weight value in optimum predictive model[2] | Weight value in predictive model[3] |
|---|---|---|---|---|
| KLF7[1] | 8609 | 16907623 | 0.3680 ** | 0.2068 |
| SNORA38B[1] | 100124536 | 16837226 | 0.2408 * | 0.1488 . |
| RAB19[1] | 401409 | 17052295 | -0.2278 | -0.0813 |
| ATP6V0D1[1] | 9114 | 16827366 | -0.1356 | -0.0783 |
| SPRY3 | 10251 | 17108597 | NA | -0.0448 |
| GPR97 | 22487 | 16819563 | NA | -0.2439 |
| SNORD116-24 | 100033435 | 16798216 | NA | 0.1092 |
| SLC43A2 | 124935 | 16839425 | NA | 0.1227 |
| ZSWIM6 | 57688 | 16985094 | NA | 0.2362 * |
| LOC399715 | 399715 | 16702215 | NA | 0.1144 |

[1] Genes belonging to the optimum subset of genes with predictive capabilities. [2] Weight value for intercept and gender is -9.4103 and 0.1652 respectively. Male coded as '1' and female coded as '0'. [3] Weight value for intercept and gender is -11.5154 and 0.1389 respectively. Same previous coding.

**Table 3**: List of 14 genes with highest predictive capabilities as identified by the iterative process applied to a PLSR model construction. Their weight values according to Eq. 2 are also shown in case of considering eight (optimum number) or fourteen genes (maximum number for obtaining a PRESS value <0.25). A p-value is indicated for each weight value coded as *** < 0.001, ** < 0.01, * < 0.05 and . < 0.1.

**Prediction versus observed categorical values of subject training level**

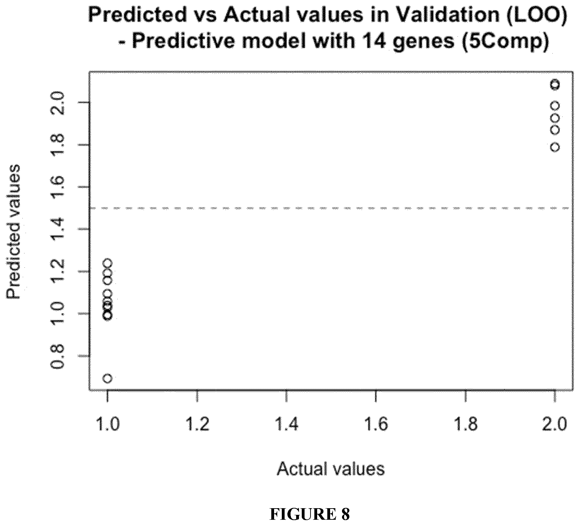

Figure 8 shows the predicted values obtained by the PLSR model constructed with the top eight genes listed in Table 3 and applied to the basal gene expression levels of the 16 available samples. These predicted values are compared to the real ones in terms of subject training level coding elite individuals with '2' and active ones with '1'.

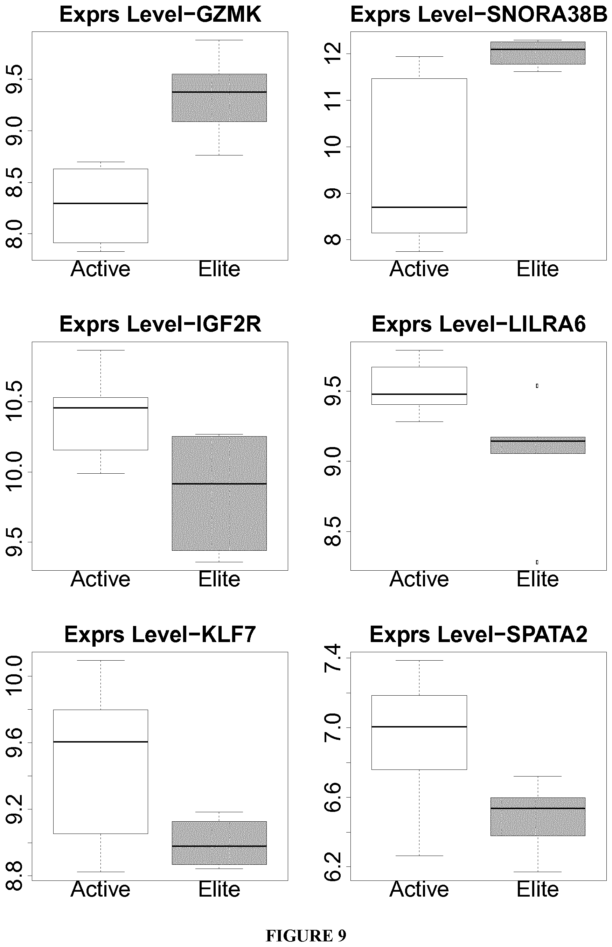

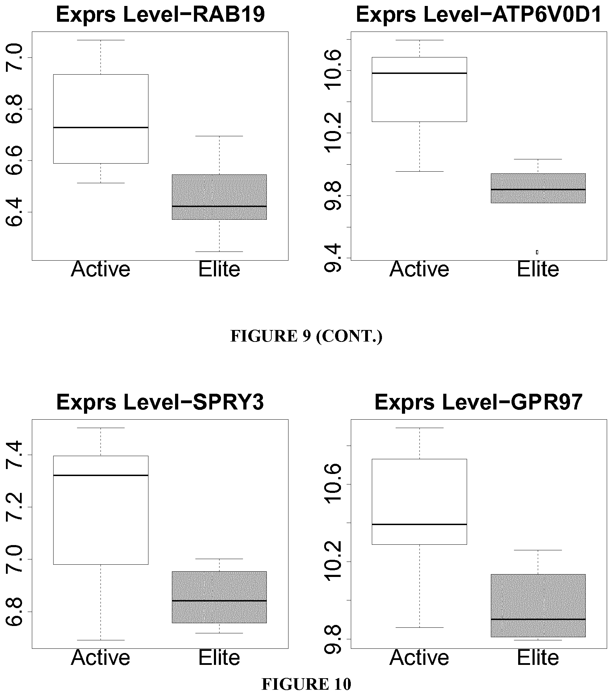

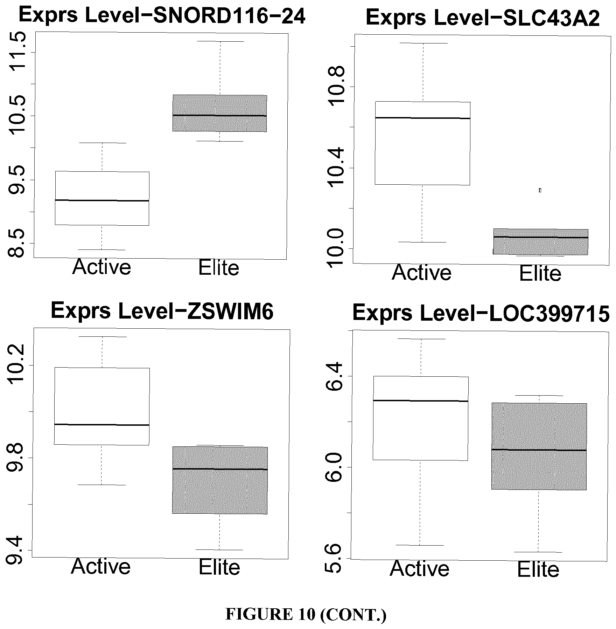

Figure 9 and 10 respectively show the log2 basal expression levels for the eight genes included in the optimum predictive model and the further set of seven genes for obtaining a predictive model with PRESS value below 0.25. These graphs considered the boxplots according to the subject fitness level.

## EXAMPLE 2

Following the same methodology as in Example 1 (blood sample, microarray platform used, and gene expression data acquisition) a new cohort of subjects was used (validation cohort).

This validation cohort was composed of a total of 20 male runners (aged 36.3 $\pm$ 8.5 years) voluntarily recruited to participate.

Experiment was deployed on the "Volta a la Cerdanya UltraFons" in 2013, with a length of approximately 35 Km and a total cumulative elevation gain was of 3930 m. The minimum altitude was of 1112 m and the Maximum altitude was of 2060 m

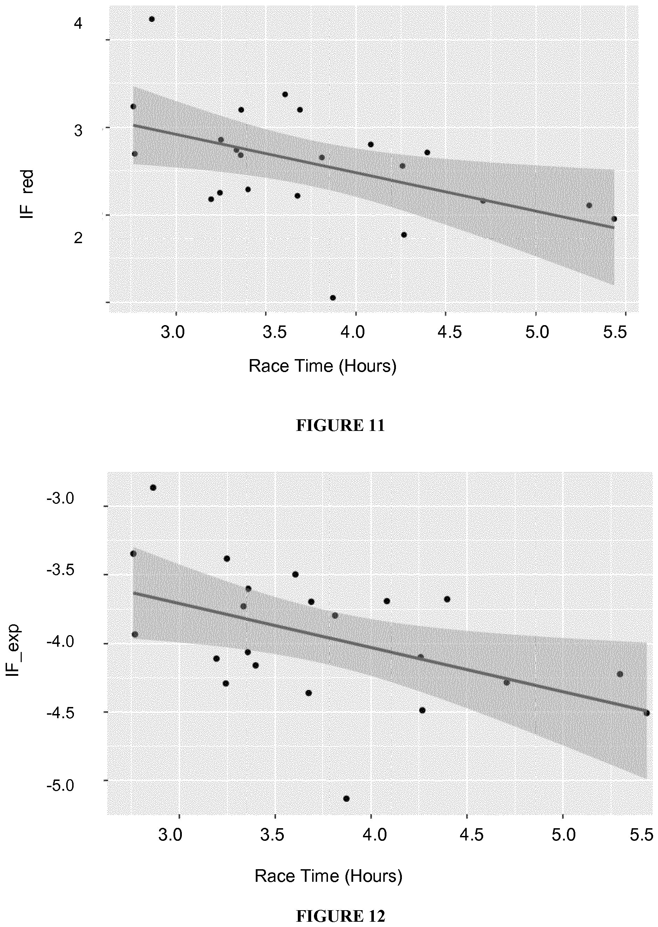

Figures 11 and 12 show correlation between the Individual Fitness (IF) value obtained from the predictive model using equation below (as in Example 1) and the final race time of each subject.

$$\text{Individual\_fitness} \approx w_0 + w_1 \cdot \text{gender} + \sum_{i=2}^{n} w_i \cdot g_i + \epsilon$$

In Figure 11 the genes used were GZMK, SNORA38B, IGF2R, LILRA6, KLF7, SPATA2, RAB19, and ATP6V0D1, showing thus an IF with a reduced number of genes (IF-red). In Figure 12 additional genes SPRY3, GPR97, SNORD116-24, SLC43A2, ZSWIM6, and LOC399715 were considered summing up to the fourteen of Table 3, and giving an IF with an expanded model (IF_exp). For each model the weight values were those of columns 4 or 5 of Table 3.

As can be seen in Figures 11 and 12 a meaningful correlation was observed (pval= 0.02017 in Figure 11, and pval=0.0227 in Figure 12).

These data corroborate that both models are good for determining fitness of a subject, since they have been checked in a validation cohort.

## Claims

1. An *in vitro* method for determining the fitness of a subject that comprises determining the expression level of at least one gene selected from the group consisting of GZMK, SNORA38B, and IGF2R in a sample from said subject, said sample being selected from blood, blood plasma and serum.

2. The *in vitro* method for determining the fitness of a subject according to claim 1 that comprises determining the expression level of GZMK, SNORA38B, and IGF2R.

3. The *in vitro* method for determining the fitness of a subject according to any one of claims 1 to 2, that comprises determining the expression level of the genes GZMK, SNORA38B, IGF2R, LILRA6, KLF7, SPATA2, RAB19, and ATP6V0D1 in a sample from said subject, said sample being selected from blood, blood plasma and serum

4. The *in vitro* method for determining the fitness of a subject according to claim 3 that comprises:

   (i) determining the expression level of the genes GZMK, SNORA38B, IGF2R, LILRA6, KLF7, SPATA2, RAB19, and ATP6V0D1 in a sample from said subject, said sample being selected from blood, blood plasma and serum; and
   (ii) calculating a predictive factor by summing the expression levels of said gene(s), wherein said expression levels are corrected by a particular coefficient for each of the gene(s),

   wherein said predictive factor is indicative of the fitness of the subject, and wherein the coefficient for each of the genes is a coefficient as shown in column 4 of Table 3.

5. The *in vitro* method according to claim 4, further comprising:

   determining the expression levels of one or more genes selected from the group consisting of SPRY3, GPR97, SNORD116-24, SLC43A2, ZSWIM6, and LOC399715 in a sample from said subject, selected from blood, blood plasma and serum; and

calculating a predictive factor by summing the expression levels of GZMK, SNORA38B, IGF2R, LILRA6, KLF7, SPATA2, RAB19, ATP6V0D1, SPRY3, GPR97, SNORD116-24, SLC43A2, ZSWIM6, and LOC399715 genes, wherein said expression levels are corrected by a particular coefficient as shown in column 5 of Table 3 for each of the genes.

6. An *in vitro* method for determining the fitness of a subject that comprises:

(i) determining the expression levels of one or more genes selected from the group consisting of GZMK, SNORA38B, and IGF2R, in a sample from said subject, said sample being selected from blood, blood plasma and serum, and

(ii) comparing the expression levels obtained in (i) with a reference value, wherein:

if the reference value is obtained from at least one subject that practices more than 10 hours a week of physical exercise, termed elite/professional subject,

- an increased or equal expression level of at least one of GZMK and SNORA38B genes compared to the reference value, and/or a decreased or equal expression level of IGF2R, gene compared to the reference value indicates that the fitness of the subject is elite/professional, and
- a decreased expression level of at least one of GZMK and SNORA38B genes compared to the reference value, and/or an increased expression level of IGF2R gene compared to the reference value indicates that the fitness of the subject is active/acceptable or passive/sedentary,

if the reference value is obtained from at least one subject that practices between three and ten hours a week of physical exercise, termed active or acceptable subject,

- a decreased or equal expression level of at least one of GZMK and SNORA38B genes compared to the reference value, and/or an increased or equal expression level of IGF2R gene compared to the reference value indicates that the fitness of the subject is active/acceptable or passive/sedentary, and
- an increased expression level of at least one of GZMK and SNORA38B genes compared to the reference value, and/or a decreased expression level of IGF2R gene compared to the reference value indicates that the fitness of the subject is elite/professional, and

if the reference value is obtained from at least one subject that practices less than three hours of physical exercise a week, termed passive/sedentary subject,

- an increased expression level of at least one of GZMK and SNORA38B genes compared to the reference value, and/or a decreased expression level of IGF2R gene compared to the reference value indicates that the fitness of the subject is elite/professional or active/acceptable, and
- a decreased or equal expression level of at least one of GZMK and SNORA38B genes compared to the reference value, and/or an increased or equal expression level of IGF2R gene compared to the reference value indicates that the fitness of the subject is passive/sedentary.

7. The *in vitro* method for determining the fitness of a subject according to claim 6 that comprises:

(i) determining the expression levels of genes GZMK, SNORA38B, IGF2R, LILRA6, KLF7, SPATA2, RAB19, and ATP6V0D1 in a sample from said subject, said sample being selected from blood, blood plasma and serum, and

(ii) comparing the expression levels obtained in (i) with a reference value, wherein:

if the reference value is obtained from one or more elite/professional subjects,

- an increased or equal expression level of at least one of GZMK and SNORA38B genes compared to the reference value, and/or a decreased or equal expression level of at least one of IGF2R, LILRA6, KLF7, SPATA2, RAB19 and ATP6V0D1 genes compared to the reference value indicates that the fitness of the subject is elite/professional, and
- a decreased expression level of at least one of GZMK and SNORA38B genes compared to the reference value, and/or an increased expression level of at least one of IGF2R, LILRA6, KLF7, SPATA2, RAB19 and ATP6V0D1 genes compared to the reference value indicates that the fitness of the subject is active/acceptable or passive/sedentary,

if the reference value is obtained from one or more active or acceptable subjects,

- a decreased or equal expression level of at least one of GZMK and SNORA38B genes compared to the reference value, and/or an increased or equal expression level of at least one of IGF2R, LILRA6, KLF7, SPATA2, RAB19 and ATP6V0D1 genes compared to the reference value indicates that the fitness of the subject is active/acceptable or passive/sedentary, and
- an increased expression level of at least one of GZMK and SNORA38B genes compared to the reference value, and/or a decreased expression level of at least one of IGF2R, LILRA6, KLF7, SPATA2, RAB19 and ATP6V0D1 genes compared to the reference value indicates that the fitness of the subject is elite/professional, and

if the reference value is obtained from one or more passive/sedentary subjects,

- an increased expression level of at least one of GZMK and SNORA38B genes compared to the reference value, and/or a decreased expression level of at least one of IGF2R, LILRA6, KLF7, SPATA2, RAB19 and ATP6V0D1 genes compared to the reference value indicates that the fitness of the subject is elite/professional or active/acceptable, and
- a decreased or equal expression level of at least one of GZMK and SNORA38B genes compared to the reference value, and/or an increased or equal expression level of at least one of IGF2R, LILRA6, KLF7, SPATA2, RAB19 and ATP6V0D1 genes compared to the reference value indicates that the fitness of the subject is passive/sedentary.

8. An *in vitro* method for selecting a subject as having a fitness level suitable to participate in an endurance sport that comprises determining the fitness of the subject using the method according to any one of claims 1 to 5 or the method according to any one of claims 7 to 8, wherein if the fitness level is active/acceptable or elite/professional the subject has a fitness level suitable to participate in an endurance sport.

9. An *in vitro* method for monitoring the response of a subject to a training program aimed at improving fitness of a subject that comprises:

(i) determining the expression levels of one or more genes selected from the group consisting of GZMK, SNORA38B, and IGF2R, in a sample from said subject, said sample being selected from blood, blood plasma and serum, during or after the training program, and
(ii) comparing the expression levels obtained in (i) with the levels of said gene(s) in a sample from the same subject, said sample being selected from blood, blood plasma and serum, in a previous point of time,

wherein an increased expression level of at least one of GZMK and SNORA38B genes, and/or a decreased expression level of IGF2R gene compared to the levels in the sample from a previous point of time indicates that the subject has a good response to training, and
wherein a decreased expression level or a lack of change of at least one of GZMK and SNORA38B genes, and/or an increased expression level or a lack of change of IGF2R gene compared to the levels in the sample from a previous point of time indicates that the subject has a bad response to training.

10. The *in vitro* method for monitoring the response of a subject to a training program aimed at improving fitness of a subject according to claim 9 that comprises:

(i) determining the expression levels of genes GZMK, SNORA38B, IGF2R, LILRA6, KLF7, SPATA2, RAB19 and ATP6V0D1 in a sample from said subject, said sample being selected from blood, blood plasma and serum, during or after the training program, and
(ii) comparing the expression levels obtained in (i) with the levels of said gene(s) in a sample from the same subject, said sample being selected from blood, blood plasma and serum, in a previous point of time,

wherein an increased expression level of at least one of GZMK and SNORA38B genes, and/or a decreased expression level of at least one of IGF2R, LILRA6, KLF7, SPATA2, RAB19 and ATP6V0D1 genes compared to the levels in the sample from a previous point of time indicates that the subject has a good response to training, and wherein a decreased expression level or a lack of change of at least one of GZMK and SNORA38B genes, and/or an increased expression level or a lack of change of at least one of IGF2R, LILRA6, KLF7, SPATA2, RAB19 and ATP6V0D1 genes compared to the levels in the sample from a previous point of time indicates that the subject has a bad response to training.

11. An *in vitro* method for selecting a personalized training program aimed at improving fitness of a subject that comprises:

(i) determining the expression levels of one or more genes selected from the group consisting of GZMK, SNORA38B, and IGF2R, in a sample from said subject, said sample being selected from blood, blood plasma and serum, during or after the training program under test, and
(ii) comparing the expression levels obtained in (i) with the levels of a sample from the same subject, said sample being selected from blood, blood plasma and serum, before starting the training program under test,

wherein the training program under test is selected if there is an increased expression level of at least one of GZMK and SNORA38B genes, and/or a decreased expression level of IGF2R gene compared to the level of expression before starting the training program, and
wherein the training program under test is not selected if there is a decreased expression level or a lack of change of at least one of GZMK and SNORA38B genes, and/or an increased expression level or a lack of change of IGF2R gene compared to the level of expression before starting the training program.

12. *The in vitro* method for selecting a personalized training program aimed at improving fitness of a subject, according to claim 14 that comprises determining the expression level of GZMK, SNORA38B and IGF2R.

13. *The in vitro* method for selecting a personalized training program aimed at improving fitness of a subject according to any one of claims 12 to 13 that comprises:

(i) determining the expression levels of genes GZMK, SNORA38B, IGF2R, LILRA6, KLF7, SPATA2, RAB19 and ATP6VOD1 in a sample from said subject, said sample being selected from blood, blood plasma and serum, during or after the training program under test, and
(ii) comparing the expression levels obtained in (i) with the levels of a sample from the same subject, said sample being selected from blood, blood plasma and serum, before starting the training program under test,

wherein the training program under test is selected if there is an increased expression level of at least one of GZMK and SNORA38B genes, and/or a decreased expression level of at least one of IGF2R, LILRA6, KLF7, SPATA2, RAB19 and ATP6VOD1 genes compared to the level of expression before starting the training program, and
wherein the training program under test is not selected if there is a decreased expression level or a lack of change of at least one of GZMK and SNORA38B genes, and/or an increased expression level or a lack of change of at least one of IGF2R, LILRA6, KLF7, SPATA2, RAB19 and ATP6VOD1 genes compared to the level of expression before starting the training program.

14. A computer-implemented method for determining the fitness of a subject according to any one of claims 1 to 7, or for selecting a subject as having a fitness level suitable to participate in an endurance sport according to claim 8, or for monitoring the response to training of a subject during an endurance exercise training according to any one of claims 9 to 10, or for selecting a personalized training program aimed at improving fitness of a subject according to any one of claims 11 to 13, which computer-implemented method comprises:

- collection of the data of the expression level of the genes determined in methods of claims 1 to 13;
- analyzing the collected data by (a) calculating a predictive factor by summing the expression levels of said genes, wherein said expression levels are corrected by a particular coefficient for each of the genes, wherein said predictive factor is indicative of the fitness of the subject, and wherein said coefficient for each of the genes is a coefficient as shown in columns 4 and 5 of Table 3; or by (b) comparing the expression levels obtained with a reference value; or by (c) comparing the expression levels obtained with the levels of said genes in a previous point of time; or by (d) comparing the expression levels with the levels of said genes, before starting a training program under test; and
- providing a result of the analysis.

15. A computer program comprising instructions in a computer readable form for implementing the computer-implemented method according to claim 14.

**Patentansprüche**

1. In-vitro-Verfahren zur Bestimmung der Fitness eines Individuums, das die Bestimmung des Expressionsniveaus mindestens eines Gens, ausgewählt aus der Gruppe bestehend aus GZMK, SNORA38B und IGF2R, in einer Probe des Individuums umfasst, wobei die Probe aus Blut, Blutplasma und Serum ausgewählt wird.

2. In-vitro-Verfahren zur Bestimmung der Fitness eines Individuums gemäß Anspruch 1, das die Bestimmung des Expressionsniveaus von GZMK, SNORA38B und IGF2R umfasst.

3. In-vitro-Verfahren zur Bestimmung der Fitness eines Individuums gemäß einem der Ansprüche 1 bis 2, das die Bestimmung des Expressionsniveaus der Gene GZMK, SNORA38B, IGF2R, LILRA6, KLF7, SPATA2, RAB19 und ATP6V0D1 in einer Probe des Individuums umfasst, wobei die Probe aus Blut, Blutplasma und Serum ausgewählt wird.

4. In-vitro-Verfahren zur Bestimmung der Fitness eines Individuums gemäß Anspruch 3, das Folgendes umfasst:

   (i) bestimmen des Expressionsniveaus der Gene GZMK, SNORA38B, IGF2R, LILRA6, KLF7, SPATA2, RAB19 und ATP6V0D1 in einer Probe des Individuums, wobei die Probe aus Blut, Blutplasma und Serum ausgewählt wird; und
   (ii) berechnen eines Vorhersagefaktors durch Summieren der Expressionsniveaus des Gens oder der Gene, wobei die Expressionsniveaus um einen bestimmten Koeffizienten für jedes der Gene korrigiert werden,

   wobei dieser Vorhersagefaktor die Fitness des Individuums anzeigt und wobei der Koeffizient für jedes der Gene ein Koeffizient ist, wie in Spalte 4 der Tabelle 3 gezeigt wird.

5. In-vitro-Verfahren gemäß Anspruch 4, ferner Folgendes umfassend:

   bestimmen der Expressionsniveaus eines oder mehrerer Gene, ausgewählt aus der Gruppe bestehend aus SPRY3, GPR97, SNORD116-24, SLC43A2, ZSWIM6 und LOC399715 in einer Probe des Individuums, ausgewählt aus Blut, Blutplasma und Serum; und
   berechnen eines Vorhersagefaktors durch Summieren der Expressionsniveaus der Gene GZMK, SNORA38B, IGF2R, LILRA6, KLF7, SPATA2, RAB19, ATP6V0D1, SPRY3, GPR97, SNORD116-24, SLC43A2, ZSWIM6 und LOC399715, wobei die Expressionsniveaus um einen bestimmten Koeffizienten korrigiert werden, wie in Spalte 5 der Tabelle 3 für jedes der Gene gezeigt wird.

6. In-vitro-Verfahren zur Bestimmung der Fitness eines Individuums, das Folgendes umfasst:

   (i) bestimmen des Expressionsniveaus eines oder mehrerer Gene, ausgewählt aus der Gruppe bestehend aus GZMK, SNORA38B und IGF2R, in einer Probe des Individuums, wobei die Probe aus Blut, Blutplasma und Serum ausgewählt wird, und
   (ii) vergleichen der in (i) gewonnenen Expressionsniveaus mit einem Referenzwert, wobei:

   wenn der Referenzwert von mindestens einem Individuum gewonnen wird, das mehr als 10 Stunden pro Woche körperlich trainiert, welches als Elite-/Profi-Individuum bezeichnet wird,

   - ein erhöhtes oder gleiches Expressionsniveau von mindestens einem der Gene GZMK und SNORA38B im Vergleich zum Referenzwert und/oder ein vermindertes oder gleiches Expressionsniveau des Gens IGF2R im Vergleich zum Referenzwert darauf hinweist, dass die Fitness des Individuums elitär/professionell ist, und
   - ein vermindertes Expressionsniveau von mindestens einem der Gene GZMK und SNORA38B im Vergleich zum Referenzwert und/oder ein erhöhtes Expressionsniveau des IGF2R-Gens im Vergleich zum Referenzwert darauf hinweist, dass die Fitness des Individuums aktiv/akzeptabel oder passiv/bewegungsarm ist,

   wenn der Referenzwert von mindestens einem Individuum gewonnen wird, das zwischen drei und zehn Stunden pro Woche körperlich trainiert, welches als aktives oder akzeptables Individuum bezeichnet wird,

- ein vermindertes oder gleiches Expressionsniveau von mindestens einem der Gene GZMK und SNORA38B im Vergleich zum Referenzwert und/oder ein erhöhtes oder gleiches Expressionsniveau des Gens IGF2R im Vergleich zum Referenzwert darauf hinweist, dass die Fitness des Individuums aktiv/akzeptabel oder passiv/bewegungsarm ist, und
- ein erhöhtes Expressionsniveau von mindestens einem der Gene GZMK und SNORA38B im Vergleich zum Referenzwert und/oder ein vermindertes Expressionsniveau des Gens IGF2R im Vergleich zum Referenzwert darauf hinweist, dass die Fitness des Individuums elitär/professionell ist, und

wenn der Referenzwert von mindestens einem Individuum gewonnen wird, das weniger als drei Stunden pro Woche körperlich trainiert, welches als passives/bewegungsarmes Individuum bezeichnet wird,

- ein erhöhtes Expressionsniveau von mindestens einem der Gene GZMK und SNORA38B im Vergleich zum Referenzwert und/oder ein vermindertes Expressionsniveau des Gens IGF2R im Vergleich zum Referenzwert darauf hinweist, dass die Fitness des Individuums elitär/professionell oder aktiv/akzeptabel ist, und
- ein vermindertes oder gleiches Expressionsniveau von mindestens einem der Gene GZMK und SNORA38B im Vergleich zum Referenzwert und/oder ein erhöhtes oder gleiches Expressionsniveau des Gens IGF2R im Vergleich zum Referenzwert darauf hinweist, dass die Fitness des Individuums passiv/bewegungsarm ist.

7. In-vitro-Verfahren zur Bestimmung der Fitness eines Individuums gemäß Anspruch 6, das Folgendes umfasst:

(i) bestimmen der Expressionsniveaus der Gene GZMK, SNORA38B, IGF2R, LILRA6, KLF7, SPATA2, RAB19 und ATP6V0D1 in einer Probe des Individuums, wobei die Probe aus Blut, Blutplasma und Serum ausgewählt wird, und
(ii) vergleichen der in (i) gewonnenen Expressionsniveaus mit einem Referenzwert,
wobei:

wenn der Referenzwert von einem oder mehreren Elite-/Profi-Individuen gewonnen wird,

- ein erhöhtes oder gleiches Expressionsniveau von mindestens einem der Gene GZMK und SNORA38B im Vergleich zum Referenzwert und/oder ein vermindertes oder gleiches Expressionsniveau von mindestens einem der Gene IGF2R, LILRA6, KLF7, SPATA2, RAB19 und ATP6V0D1 im Vergleich zum Referenzwert darauf hinweist, dass die Fitness des Individuums elitär/professionell ist, und
- ein vermindertes Expressionsniveau von mindestens einem der Gene GZMK und SNORA38B im Vergleich zum Referenzwert und/oder ein erhöhtes Expressionsniveau von mindestens einem der Gene IGF2R, LILRA6, KLF7, SPATA2, RAB19 und ATP6V0D1 im Vergleich zum Referenzwert darauf hinweist, dass die Fitness des Individuums aktiv/akzeptabel oder passiv/bewegungsarm ist,

wenn der Referenzwert von einem oder mehreren aktiven oder akzeptablen Individuen gewonnen wird,

- ein vermindertes oder gleiches Expressionsniveau von mindestens einem der Gene GZMK und SNORA38B im Vergleich zum Referenzwert und/oder ein erhöhtes oder gleiches Expressionsniveau von mindestens einem der Gene IGF2R, LILRA6, KLF7, SPATA2, RAB19 und ATP6V0D1 im Vergleich zum Referenzwert darauf hinweist, dass die Fitness des Individuums aktiv/akzeptabel oder passiv/bewegungsarm ist, und
- ein erhöhtes Expressionsniveau von mindestens einem der Gene GZMK und SNORA38B im Vergleich zum Referenzwert und/oder ein vermindertes Expressionsniveau von mindestens einem der Gene IGF2R, LILRA6, KLF7, SPATA2, RAB19 und ATP6V0D1 im Vergleich zum Referenzwert darauf hinweist, dass die Fitness des Individuums elitär/professionell ist, und

wenn der Referenzwert von einem oder mehreren passiven/bewegungsarmen Individuen gewonnen wird,

- ein erhöhtes Expressionsniveau von mindestens einem der Gene GZMK und SNORA38B im Vergleich zum Referenzwert und/oder ein vermindertes Expressionsniveau von mindestens einem der Gene IGF2R, LILRA6, KLF7, SPATA2, RAB19 und ATP6V0D1 im Vergleich zum Referenzwert darauf hinweist, dass die Fitness des Individuums elitär/professionell oder aktiv/akzeptabel ist, und
- ein vermindertes oder gleiches Expressionsniveau von mindestens einem der Gene GZMK und SNORA38B im Vergleich zum Referenzwert und/oder ein erhöhtes oder gleiches Expressionsniveau von mindestens einem der Gene IGF2R, LILRA6, KLF7, SPATA2, RAB19 und ATP6V0D1 im Vergleich zum Referenzwert darauf hinweist, dass die Fitness des Individuums passiv/bewegungsarm ist.

8. In-vitro-Verfahren zum Auswählen eines Individuums, das ein Fitnessniveau aufweist, das zur Teilnahme an einem Ausdauersport geeignet ist, das die Bestimmung der Fitness des Individuums unter Verwendung des Verfahrens gemäß einem der Ansprüche 1 bis 5 oder des Verfahrens gemäß einem der Ansprüche 7 bis 8 umfasst, wobei das Individuum, wenn das Fitnessniveau aktiv/akzeptabel oder elitär/professionell ist, ein Fitnessniveau aufweist, das zur Teilnahme an einem Ausdauersport geeignet ist.

9. In-vitro-Verfahren zur Überwachung der Reaktion eines Individuums auf ein Trainingsprogramm, das darauf abzielt, die Fitness eines Individuums zu verbessern, das Folgendes umfasst:

(i) bestimmen des Expressionsniveaus eines oder mehrerer Gene, ausgewählt aus der Gruppe bestehend aus GZMK, SNORA38B und IGF2R, in einer Probe des Individuums, wobei die Probe aus Blut, Blutplasma und Serum ausgewählt wird, während oder nach dem Trainingsprogramm, und
(ii) vergleichen der in (i) gewonnenen Expressionsniveaus mit den Niveaus des Gens oder der Gene in einer Probe desselben Individuums, wobei die Probe aus Blut, Blutplasma und Serum ausgewählt wird, zu einem früheren Zeitpunkt,

wobei ein erhöhtes Expressionsniveau von mindestens einem der Gene GZMK und SNORA38B und/oder ein vermindertes Expressionsniveau des Gens IGF2R im Vergleich zu den Niveaus in der Probe von einem früheren Zeitpunkt darauf hinweist, dass das Individuum eine gute Reaktion auf das Training aufweist, und wobei ein vermindertes Expressionsniveau oder eine fehlende Veränderung von mindestens einem der Gene GZMK und SNORA38B und/oder ein erhöhtes Expressionsniveau oder eine fehlende Veränderung des IGF2R-Gens im Vergleich zu den Niveaus in der Probe von einem früheren Zeitpunkt darauf hinweist, dass das Individuum eine schlechte Reaktion auf das Training aufweist.

10. In-vitro-Verfahren zur Überwachung der Reaktion eines Individuums auf ein Trainingsprogramm, das darauf abzielt, die Fitness eines Individuums zu verbessern, gemäß Anspruch 9, das Folgendes umfasst:

(i) bestimmen der Expressionsniveaus der Gene GZMK, SNORA38B, IGF2R, LILRA6, KLF7, SPATA2, RAB19 und ATP6V0D1 in einer Probe des Individuums, wobei die Probe aus Blut, Blutplasma und Serum ausgewählt wird, während oder nach dem Trainingsprogramm, und
(ii) vergleichen der in (i) gewonnenen Expressionsniveaus mit den Niveaus des Gens oder der Gene in einer Probe desselben Individuums, wobei die Probe aus Blut, Blutplasma und Serum ausgewählt wird, zu einem früheren Zeitpunkt,

wobei ein erhöhtes Expressionsniveau von mindestens einem der Gene GZMK und SNORA38B und/oder ein vermindertes Expressionsniveau von mindestens einem der Gene IGF2R, LILRA6, KLF7, SPATA2, RAB19 und ATP6V0D1 im Vergleich zu den Niveaus in der Probe von einem früheren Zeitpunkt darauf hinweist, dass das Individuum eine gute Reaktion auf das Training aufweist, und wobei ein vermindertes Expressionsniveau oder eine fehlende Veränderung von mindestens einem der Gene GZMK und SNORA38B und/oder ein erhöhtes Expressionsniveau oder eine fehlende Veränderung von mindestens einem der Gene IGF2R, LILRA6, KLF7, SPATA2, RAB19 und ATP6V0D1 im Vergleich zu den Niveaus in der Probe von einem früheren Zeitpunkt darauf hinweist, dass das Individuum eine schlechte Reaktion auf das Training aufweist.

11. In-vitro-Verfahren zum Auswählen eines personalisierten Trainingsprogramms, das darauf abzielt, die Fitness eines Individuums zu verbessern, das Folgendes umfasst:

(i) bestimmen des Expressionsniveaus eines oder mehrerer Gene, ausgewählt aus der Gruppe bestehend aus GZMK, SNORA38B und IGF2R, in einer Probe des Individuums, wobei die Probe aus Blut, Blutplasma und Serum ausgewählt wird, während oder nach dem zu testenden Trainingsprogramm, und
(ii) vergleichen der in (i) gewonnenen Expressionsniveaus mit den Niveaus einer Probe desselben Individuums, wobei die Probe aus Blut, Blutplasma und Serum ausgewählt wird, bevor das zu testende Trainingsprogramm begonnen wird,

wobei das zu testende Trainingsprogramm ausgewählt wird, wenn ein erhöhtes Expressionsniveau von mindestens einem der Gene GZMK und SNORA38B und/oder ein vermindertes Expressionsniveau des Gens IGF2R im Vergleich zu dem Expressionsniveau vor Beginn des Trainingsprogramms vorliegt, und wobei das zu testende Trainingsprogramm nicht ausgewählt wird, wenn ein vermindertes Expressionsniveau oder eine fehlende Veränderung von mindestens einem der Gene GZMK und SNORA38B und/oder ein erhöhtes Expressionsniveau oder eine fehlende Veränderung des Gens IGF2R im Vergleich zu dem Expressionsniveau vor Beginn des Trainingsprogramms vorliegt.

12. In-vitro-Verfahren zum Auswählen eines personalisierten Trainingsprogramms, das darauf abzielt, die Fitness eines Individuums zu verbessern, gemäß Anspruch 14, das die Bestimmung des Expressionsniveaus von GZMK, SNORA38B und IGF2R umfasst.

13. In-vitro-Verfahren zum Auswählen eines personalisierten Trainingsprogramms, das darauf abzielt, die Fitness eines Individuums zu verbessern, gemäß einem der Ansprüche 12 bis 13, das Folgendes umfasst:

(i) bestimmen der Expressionsniveaus der Gene GZMK, SNORA38B, IGF2R, LILRA6, KLF7, SPATA2, RAB19 und ATP6V0D1 in einer Probe des Individuums, wobei die Probe aus Blut, Blutplasma und Serum ausgewählt wird, während oder nach dem zu testenden Trainingsprogramm, und
(ii) vergleichen der in (i) gewonnenen Expressionsniveaus mit den Niveaus einer Probe desselben Individuums, wobei die Probe aus Blut, Blutplasma und Serum ausgewählt wird, bevor das zu testende Trainingsprogramm begonnen wird,

wobei das zu testende Trainingsprogramm ausgewählt wird, wenn ein erhöhtes Expressionsniveau von mindestens einem der Gene GZMK und SNORA38B und/oder ein vermindertes Expressionsniveau von mindestens einem der Gene IGF2R, LILRA6, KLF7, SPATA2, RAB19 und ATP6V0D1 im Vergleich zu dem Expressionsniveau vor Beginn des Trainingsprogramms vorliegt, und wobei das zu testende Trainingsprogramm nicht ausgewählt wird, wenn ein vermindertes Expressionsniveau oder eine fehlende Veränderung von mindestens einem der Gene GZMK und SNORA38B und/oder ein erhöhtes Expressionsniveau oder eine fehlende Veränderung von mindestens einem der Gene IGF2R, LILRA6, KLF7, SPATA2, RAB19 und ATP6V0D1 im Vergleich zu dem Expressionsniveau vor Beginn des Trainingsprogramms vorliegt.

14. Computerimplementiertes Verfahren zur Bestimmung der Fitness eines Individuums gemäß einem der Ansprüche 1 bis 7, oder zum Auswählen eines Individuums, das ein Fitnessniveau aufweist, das zur Teilnahme an einem Ausdauersport geeignet ist, gemäß Anspruch 8, oder zum Überwachen der Reaktion eines Individuums auf ein Training während einer Ausdauerübung gemäß einem der Ansprüche 9 bis 10, oder zum Auswählen eines personalisierten Trainingsprogramms, das darauf abzielt, die Fitness eines Individuums gemäß einem der Ansprüche 11 bis 13 zu verbessern, wobei das computerimplementierte Verfahren Folgendes umfasst:

- sammeln der Daten des Expressionsniveaus der Gene, die in den Verfahren der Ansprüche 1 bis 13 bestimmt werden;
- analysieren der gesammelten Daten durch (a) Berechnen eines Vorhersagefaktors durch Summieren der Expressionsniveaus der Gene, wobei die Expressionsniveaus um einen bestimmten Koeffizienten für jedes der Gene korrigiert werden, wobei der Vorhersagefaktor die Fitness des Individuums anzeigt und wobei der Koeffizient für jedes der Gene ein Koeffizient ist, wie in den Spalten 4 und 5 der Tabelle 3 gezeigt wird; oder durch (b) Vergleichen der gewonnenen Expressionsniveaus mit einem Referenzwert; oder durch (c) Vergleichen der gewonnenen Expressionsniveaus mit den Niveaus der Gene zu einem früheren Zeitpunkt; oder durch (d) Vergleichen der Expressionsniveaus mit den Niveaus der Gene, bevor ein zu testendes Trainingsprogramm begonnen wird; und
- bereitstellen eines Ergebnisses der Analyse.

15. Computerprogramm, das Anweisungen in einer computerlesbaren Form umfasst, um das computerimplementierte Verfahren gemäß Anspruch 14 zu implementieren.

**Revendications**

1. Une méthode *in vitro* pour déterminer la forme physique d'un sujet qui comporte déterminer le niveau d'expression d'au moins un gène sélectionné du groupe consistant en GZMK, SNORA38B et IGF2R dans un échantillon de ce sujet, cet échantillon étant sélectionné de sang, plasma sanguin et sérum.

2. La méthode *in vitro* pour déterminer la forme physique d'un sujet conformément à la revendication 1 qui comporte déterminer le niveau d'expression de GZMK, SNORA38B et IGF2R.

3. La méthode *in vitro* pour déterminer la forme physique d'un sujet conformément à une quelconque des revendications 1 à 2 qui comporte déterminer le niveau d'expression des gènes GZMK, SNORA38B, IGF2R, LILRA6, KLF7, SPATA2, RAB19 et ATP6V0D1 dans un échantillon de ce sujet, cet échantillon étant sélectionné de sang, plasma sanguin et sérum.

4. La méthode *in vitro* pour déterminer la forme physique d'un sujet conformément à la revendication 3 comportant :

   (i) déterminer le niveau d'expression des gènes GZMK, SNORA38B, IGF2R, LILRA6, KLF7, SPATA2, RAB19 et ATP6V0D1 dans un échantillon de ce sujet, cet échantillon étant sélectionné de sang, plasma sanguin et sérum ; et
   (ii) calculer un facteur prédictif en additionnant les niveaux d'expression de ce(s) gène(s), où ces niveaux d'expression sont corrigés par un coefficient particulier pour chacun des gènes

   où ce facteur prédictif est indicatif de la forme physique du sujet et où le coefficient pour chacun des gènes est un coefficient tel que représenté dans la colonne 4 de la Table 3.

5. La méthode *in vitro* conformément à la revendication 4, comportant en outre :

   déterminer les niveaux d'expression d'un ou de plusieurs gènes sélectionnés du groupe consistant en SPRY3, GPR97, SNORD116-24, SLC43A2, ZSWIM6 et LOC399715 dans un échantillon de ce sujet sélectionné de sang, plasma sanguin et sérum ; et
   calculer un facteur prédictif en additionnant les niveaux d'expression des gènes GZMK, SNORA38B, IGF2R, LILRA6, KLF7, SPATA2, RAB19, ATP6V0D1, SPRY3, GPR97, SNORD116-24, SLC43A2, ZSWIM6 et LOC399715, où ces niveaux d'expression sont corrigés par un coefficient particulier tel que représenté dans la colonne 5 de la Table 3 pour chacun des gènes.

6. Une méthode *in vitro* pour déterminer la forme physique d'un sujet qui comporte :

   (i) déterminer les niveaux d'expression d'un ou de plusieurs gènes sélectionnés du groupe consistant en GZMK, SNORA38B et IGF2R dans un échantillon de ce sujet, cet échantillon étant sélectionné de sang, plasma sanguin et sérum, et
   (ii) comparer les niveaux d'expression obtenus dans (i) avec une valeur de référence,
   où

   si la valeur de référence est obtenue d'au moins un sujet qui pratique plus de 10 heures hebdomadaires d'exercice physique, dénommé sujet d'élite/professionnel,

   - un niveau d'expression augmenté ou égal d'au moins d'un des gènes GZMK et SNORA38B comparé à la valeur de référence, et/ou un niveau d'expression diminué ou égal de gène IGF2R comparé à la valeur de référence indique que la forme physique du sujet est d'élite/professionnelle, et
   - un niveau d'expression diminué d'au moins un des gènes GZMK et SNORA38B comparé à la valeur de référence et/ou à un niveau d'expression augmenté de gène IGF2R comparé à la valeur de référence indique que la forme physique du sujet est active/acceptable ou passive/sédentaire,

   si la valeur de référence est obtenue d'au moins un sujet qui pratique de trois à dix heures hebdomadaires d'exercice physique, dénommé un sujet actif ou acceptable,

   - un niveau d'expression diminué ou égal d'au moins un des gènes GZMK et SNORA38B comparé à la valeur de référence, et/ou un niveau d'expression augmenté ou égal de gène IGF2R comparé à la valeur de référence indique que la forme physique du sujet est active/acceptable ou passive/sédentaire, et

- un niveau d'expression augmenté d'au moins un des gènes GZMK et SNORA38B comparé à la valeur de référence et/ou un niveau d'expression diminué de gène IGF2R comparé à la valeur de référence indique que la forme physique du sujet est d'élite/professionnelle, et

si la valeur de référence est obtenue d'au moins un sujet qui pratique moins de trois heures hebdomadaires d'exercice physique, dénommé sujet passif/sédentaire

- un niveau d'expression augmenté d'au moins un des gènes GZMK et SNORA38B comparé à la valeur de référence, et/ou un niveau d'expression diminué de gène IGF2R comparé à la valeur de référence indique que la forme physique du sujet est d'élite/professionnelle ou active/acceptable, et
- un niveau d'expression diminué ou égal d'au moins un des gènes GZMK et SNORA38B comparé à la valeur de référence et/ou un niveau d'expression augmenté ou égal de gène IGF2R comparé à la valeur de référence indique que la forme physique du sujet est passive/sédentaire,

7. La méthode *in vitro* pour déterminer la forme physique d'un sujet conformément à la revendication 6 comportant :

(i) déterminer les niveaux d'expression des gènes GZMK, SNORA38B, IGF2R, LILRA6, KLF7, SPATA2, RAB19 et ATP6V0D1 dans un échantillon de ce sujet, cet échantillon étant sélectionné de sang, plasma sanguin et sérum ; et
(ii) comparer les niveaux d'expression obtenus dans (i) avec une valeur de référence, où

si la valeur de référence est obtenue d'un ou plusieurs sujets d'élite/professionnels,

- un niveau d'expression augmenté ou égal d'au moins un des gènes GZMK et SNORA38B comparé à la valeur de référence, et/ou un niveau d'expression diminué ou égal d'au moins un des gènes IGF2R, LILRA6, KLF7, SPATA2, RAB19 et ATP6V0D1 comparé à la valeur de référence indique que la forme physique de ce sujet est d'élite/professionnelle, et
- un niveau d'expression diminué d'au moins d'un des gènes GZMK et SNORA38B comparé à la valeur de référence, et/ou un niveau d'expression augmenté d'au moins un des gènes IGF2R, LILRA6, KLF7, SPATA2, RAB19 et ATP6V0D1 comparé à la valeur de référence indique que la forme physique de ce sujet est active/acceptable ou passive/sédentaire,

si la valeur de référence est obtenue d'un ou plusieurs sujets actifs ou acceptables,

- un niveau d'expression diminué ou égal d'au moins un des gènes GZMK et SNORA38B comparé à la valeur de référence, et/ou un niveau d'expression augmenté ou égal d'au moins un des gènes IGF2R, LILRA6, KLF7, SPATA2, RAB19 et ATP6V0D1 comparé à la valeur de référence indique que la forme physique de ce sujet est active/acceptable, ou passive/sédentaire, et
- un niveau d'expression augmenté d'au moins un des gènes GZMK et SNORA38B comparé à la valeur de référence, et/ou un niveau d'expression diminué d'au moins un des gènes IGF2R, LILRA6, KLF7, SPATA2, RAB19 et ATP6V0D1 comparé à la valeur de référence indique que la forme physique de ce sujet est d'élite/professionnelle et

si la valeur de référence est obtenue d'un ou plusieurs sujets passifs/sédentaires,si la valeur de référence est obtenue d'un ou plusieurs sujets passifs/sédentaires,

- un niveau d'expression augmenté d'au moins un des gènes GZMK et SNORA38B comparé à la valeur de référence, et/ou un niveau d'expression diminué d'au moins un des gènes IGF2R, LILRA6, KLF7, SPATA2, RAB19 et ATP6V0D1 comparé à la valeur de référence indique que la forme physique de ce sujet est d'élite/professionnelle ou active/acceptable, et
- un niveau d'expression diminué ou égal d'au moins un des gènes GZMK et SNORA38B comparé à la valeur de référence, et/ou un niveau d'expression augmenté ou égal d'au moins un des gènes IGF2R, LILRA6, KLF7, SPATA2, RAB19 et ATP6V0D1 comparé à la valeur de référence indique que la forme physique de ce sujet est passive/sédentaire.

8. Une méthode in *vitro* pour sélectionner un sujet ayant un niveau de forme physique approprié pour participer à un sport d'endurance qui comporte déterminer la forme physique du sujet en utilisant la méthode conformément à une quelconque des revendications 1 à 5 ou la méthode conformément à une quelconque des revendications 7 à 8, où, si le niveau de forme physique est active/acceptable ou d'élite/professionnelle, le sujet possède un niveau de forme physique approprié pour participer à un sport d'endurance.

9. Une méthode in *vitro* pour le monitoring de la réponse d'un sujet à un programme d'entraînement ayant pour but améliorer la forme physique d'un sujet comportant :

   (i) déterminer les niveaux d'expression d'un ou plusieurs gènes sélectionnés du groupe consistant en GZMK, SNORA38B et IGF2R dans un échantillon de ce sujet, cet échantillon étant sélectionné de sang, plasma sanguin et sérum, durant ou après le programme d'entraînement, et
   (ii) comparer les niveaux d'expression obtenus dans (i) avec les niveaux de ce(s) gène(s) dans un échantillon du même sujet, cet échantillon étant sélectionné de sang, plasma sanguin et sérum, à un moment donné précédent,

   où un niveau d'expression augmenté d'au moins un des gènes GZMK et SNORA38B et/ou un niveau d'expression diminué du gène IGF2R comparé aux niveaux dans l'échantillon d'un moment donné précédent indique que le sujet possède une bonne réponse à l'entraînement et
   où un niveau d'expression diminué ou un manque de changement d'au moins un des gènes GZMK et SNORA38B et/ou un niveau d'expression augmenté ou un manque de changement du gène IGF2R comparé aux niveaux dans l'échantillon d'un moment donné précédent indique que le sujet possède une mauvaise réponse à l'entraînement.

10. La méthode in *vitro* pour le monitoring de la réponse d'un sujet à un programme d'entraînement ayant pour but améliorer la forme physique d'un sujet, conformément à la revendication 9 comportant :

    (i) déterminer les niveaux d'expression des gènes GZMK, SNORA38B, IGF2R LILRA6, KLF7, SPATA2, RAB19 et ATP6V0D1 dans un échantillon de ce sujet, cet échantillon étant sélectionné de sang, plasma sanguin et sérum, durant ou après le programme d'entraînement, et
    (ii) comparer les niveaux d'expression obtenus dans (i) avec les niveaux de ce(s) gène(s) dans un échantillon du même sujet, cet échantillon étant sélectionné de sang, plasma sanguin et sérum, à un moment donné précédent,

    où un niveau d'expression augmenté d'au moins un des gènes GZMK et SNORA38B et/ou un niveau d'expression diminué d'au moins un des gènes IGF2R LILRA6, KLF7, SPATA2, RAB19 et ATP6V0D1 comparé aux niveaux dans l'échantillon d'un moment donné précédent indique que le sujet possède une bonne réponse à l'entraînement et où un niveau d'expression diminué ou un manque de changement d'au moins un des gènes GZMK et SNORA38B et/ou un niveau d'expression augmenté ou un manque de changement d'au moins un des gènes IGF2R LILRA6, KLF7, SPATA2, RAB19 et ATP6V0D1 comparé aux niveaux dans l'échantillon d'un moment donné précédent indique que le sujet possède une mauvaise réponse à l'entraînement.

11. Une méthode in *vitro* pour sélectionner un programme d'entraînement personnalisé ayant pour but améliorer la forme physique d'un sujet comportant :

    (i) déterminer les niveaux d'expression d'un ou plusieurs gènes sélectionnés du groupe consistant en GZMK, SNORA38B et IGF2R dans un échantillon de ce sujet, cet échantillon étant sélectionné de sang, plasma sanguin et sérum, durant ou après le programme d'entraînement qui est testé, et
    (ii) comparer les niveaux d'expression obtenus dans (i) avec les niveaux d'un échantillon du même sujet, cet échantillon étant sélectionné de sang, plasma sanguin et sérum, avant de commencer le programme d'entraînement qui est testé,

    où le programme d'entraînement qui est testé est sélectionné s'il y a un niveau d'expression augmenté d'au moins un des gènes GZMK et SNORA38B et/ou un niveau d'expression diminué du gène IGF2R comparé au niveau d'expression avant de commencer le programme d'entraînement, et
    où le programme d'entraînement qui est testé n'est pas sélectionné s'il y a un niveau d'expression diminué ou un manque de changement d'au moins un des gènes GZMK et SNORA38B, et/ou un niveau d'expression augmenté ou un manque de changement du gène IGF2R comparé au niveau d'expression avant de commencer le programme d'entraînement.

**12.** La méthode in *vitro* pour sélectionner un programme d'entraînement personnalisé ayant pour but améliorer la forme physique d'un sujet, conformément à la revendication 14 qui comporte déterminer le niveau d'expression de GZMK, SNORA38B et IGF2R.

**13.** La méthode in *vitro* pour sélectionner un programme d'entraînement personnalisé ayant pour but améliorer la forme physique d'un sujet, conformément à une quelconque des revendications 12 à 13 comportant :

(i) déterminer les niveaux d'expression des gènes GZMK, SNORA38B, IGF2R, LILRA6, KLF7, SPATA2, RAB19 et ATP6V0D1 dans un échantillon de ce sujet, cet échantillon étant sélectionné de sang, plasma sanguin et sérum, durant ou après le programme d'entraînement qui est testé, et
(ii) comparer les niveaux d'expression obtenus dans (i) avec les niveaux d'un échantillon du même sujet, cet échantillon étant sélectionné de sang, plasma sanguin et sérum, avant de commencer le programme d'entraînement qui est testé ;

où le programme d'entraînement qui est testé est sélectionné s'il y a un niveau d'expression augmenté d'au moins un des gènes GZMK et SNORA38B et/ou un niveau d'expression diminué d'au moins un des gènes IGF2R, LILRA6, KLF7, SPATA2, RAB19 et ATP6V0D1 comparé au niveau d'expression avant de commencer le programme d'entraînement, et
où le programme d'entraînement n'est pas sélectionné s'il y a un niveau d'expression diminué ou un manque de changement d'au moins un des gènes GZMK et SNORA38B et/ou un niveau d'expression augmenté ou un manque de changement d'au moins un des gènes IGF2R, LILRA6, KLF7, SPATA2, RAB19 et ATP6V0D1, comparé au niveau d'expression avant de commencer le programme d'entraînement,

**14.** Une méthode mise en œuvre par ordinateur pour déterminer la forme physique d'un sujet conformément à une quelconque des revendications 1 à 7 ou pour sélectionner un sujet comme ayant un niveau de forme physique approprié pour participer dans un sport d'endurance conformément à la revendication 8 ou pour le monitoring de la réponse à l'entraînement d'un sujet durant un entraînement d'exercice d'endurance conformément à une quelconque des revendications 9 à 10, ou pour sélectionner un programme d'entraînement personnalisé ayant pour but améliorer la forme physique d'un sujet conformément à une quelconque des revendications 11 à 13, cette méthode mise en œuvre par ordinateur comportant :

- la collecte de données du niveau d'expression des gènes déterminé dans les méthodes des revendications 1 à 13 ;
- analyser les données collectées par (a) le calcul d'un facteur prédictif en additionnant les niveaux d'expression de ces gènes, où ces niveaux d'expression sont corrigés par un coefficient particulier pour chacun des gènes où ce facteur prédictif est indicatif de la forme physique du sujet et où ce coefficient pour chacun des gènes est un coefficient tel que représenté dans les colonnes 4 et 5 de la Table 3 ; où par (b) la comparaison des niveaux d'expression obtenus avec une valeur de référence ; ou par (c) la comparaison des niveaux d'expression obtenus avec les niveaux de ces gènes à un moment donné précédent ; ou par (d) la comparaison de niveaux d'expression avec les niveaux de ces gènes avant de commencer le programme d'entraînement qui est testé ; et
- offrir un résultat de l'analyse.

**15.** Un programme informatique comportant des instructions dans un ordinateur sous forme lisible pour la mise en œuvre de la méthode mise en œuvre par ordinateur conformément à la revendication 14.

| Gene expression profile of the subject before and after the race | Construction of multivariate linear model regression for modelling gene expression levels | Genes ranking based on their significant statistically differential expression | List of genes obtained from the interaction effect between training regime and measured time point (pre or post race) | Construction of a predictive model of the subject fitness | Identification of the minimum number of genes to be included in the predictive model |

**FIGURE 1**

Input data to for predictive model

Differential genes ranked obtained through the interaction effect variable in LM (eq. 1)

PLSR model construction (eq. 2) with basal gene expression levels and gender adjustment

Identification of the gene with the highest coefficient in absolute value. This gene is considered as the initial set of genes of the predictive model.

Initial set: 1 gene
Candidates list with rest of differential genes

Predictive model construction

Main loop. Number of iterations: length of initial candidates list

Secondary loop: Sweep candidate genes

Add a new candidate gen to the former set of genes to build a new PLSR model.

PRESS value monitoring and storage of the PLSR model through Leave One Out cross-validation.

Determine the PLSR model with the minimum PRESS value among all built models in secondary loop.

Identify the candidate gene which was added to the selected PLSR model

Identified candidate gene takes part of the set of genes of the PLSR model in construction. Gene is removed from list of candidate genes

Minimum set of genes in the predictive model

Identify the minimum gene set with predictive capability based on model PRESS value.

**FIGURE 2**

**PCA - Pre vs Post athletes considering their training level**

**FIGURE 3**

**Scores PC1 - From PrePost & Training ranking - PRE vs POST**

**FIGURE 4**

**PLSR iterative model - PRESS evolution as a function of the number of genes in the PLSR**

FIGURE 5

**FIG. 6**

FIGURE 7

**Predicted vs Actual values in Validation (LOO) - Predictive model with 14 genes (5Comp)**

**FIGURE 8**

FIGURE 9

## Exprs Level−RAB19

## Exprs Level−ATP6V0D1

**FIGURE 9 (CONT.)**

## Exprs Level−SPRY3

## Exprs Level−GPR97

**FIGURE 10**

## Exprs Level−SNORD116−24

## Exprs Level−SLC43A2

## Exprs Level−ZSWIM6

## Exprs Level−LOC399715

**FIGURE 10 (CONT.)**

**FIGURE 11**

**FIGURE 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20140094381 A

### Non-patent literature cited in the description

- **BRAY, M. S. et al.** *Medicine & Science in Sports & Exercise,* 2009, vol. 41 (1), 34-72
- **YOSHIOKA, M. et al.** *FASEB Journal,* 2003, vol. 17 (13), 1812-19
- **STEPTO, N. K. et al.** *Medicine & Science in Sports & Exercise,* 2009, vol. 41 (3), 546-65
- **BARREY et al.** *Gene expression profiling in blood cells of endurance horses completing competition or disqualified due to metabolic disorder*
- **GRAZYNA JANIKOWSKA et al.** *Differences in echocardiography, blood pressure, stroke volume, maximal power and profile of genes related to cardiac hypertrophy in elite road cyclists*
- **YOSHIOKA et al.** *Serial analysis of gene expression in the skeletal muscle of endurance athletes compared to sedentary men*
- **MUSTELIN et al.** *Acquired obesity and poor physical fitness impair expression of genes of mitochondrial oxidative phosphorylation in monozygotic twins discordant for obesity*
- **TIMMONS, J. A. et al.** *Journal of Applied Physiology,* 1985, vol. 108, 1487-96
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **TEAM, R. C.** *R: A language and environment for statistical computing. R Foundation for Statistical Computing.,* 2015
- **GENTLEMAN, R. C. et al.** *Genome Biol,* 2004, vol. 5 (10), R80
- **IRIZARRY, R. A. et al.** *Biostatistics,* 2003, vol. 4 (2), 249-264
- **CARVALHO, B. S. ; IRIZARRY, R. A.** *Bioinformatics,* 2010, vol. 26 (19), 2363-2367
- **BOLSTAD, B. M. et al.** *Int. Rev. Neurobiol,* 2004, vol. 60, 25-58
- **BOURGON, R.** *Proc. Natl. Acad. Sci.,* 2010, vol. 107 (21), 9546-9551
- **RITCHIE, M. E.** *Nucleic Acids Res,* 2015, vol. 43 (7), e47
- **BJØRN-HELGE MEVIK et al.** *pls: Partial Least Squares and Principal Component regression.,* 2013
- **H. MARTENS ; M. MARTENS.** *Food Qual. Prefer.,* 2000, vol. 11 (1-2), 5-16